# EUROPEAN PATENT SPECIFICATION

(11) **EP 2 389 195 B1**
(45) Date of publication and mention of the grant of the patent: **20.05.2015**
(21) Application number: 10733825.3
(22) Date of filing: 20.01.2010
(51) Int. Cl.: A61K 39/395, C07K 16/18, A61F 2/28, C07K 16/22, A61P 19/08

(54) **ANTIBODY MEDIATED OSSEOUS REGENERATION**
ANTIKÖRPERVERMITTELTE KNOCHENREGENERATION
RÉGÉNÉRATION OSSEUSE À MÉDIATION PAR DES ANTICORPS

(30) Priority: 20.01.2009 US 145963 P
(43) Date of publication of application: 30.11.2011
(73) Proprietor: Zadeh, Homayoun H., Calabasas, CA 91302 (US)
(72) Inventor: Zadeh, Homayoun H., Calabasas, CA 91302 (US)
(74) Representative: Potter Clarkson LLP
(86) International application number: PCT/US2010/021548
(87) International publication number: WO 2010/085510

(56) References cited:
- WO-A1-2010/000061
- WO-A2-2008/030611
- US-A1- 2007 154 563
- US-A1- 2008 241 108
- SACHSE A ET AL: "Osteointegration of hydroxyapatite-titanium implants coated with nonglycosylated recombinant human bone morphogenetic protein-2 (BMP-2) in aged sheep", BONE, PERGAMON PRESS., OXFORD, GB, vol. 37, no. 5, 1 November 2005 (2005-11-01), pages 699-710, XP027874586, ISSN: 8756-3282 [retrieved on 2005-11-01]
- FREIRE MARCELO O ET AL: "Antibody-mediated osseous regeneration: a novel strategy for bioengineering bone by immobilized anti-bone morphogenetic protein-2 antibodies.", TISSUE ENGINEERING. PART A DEC 2011, vol. 17, no. 23-24, December 2011 (2011-12), pages 2911-2918, XP002687314, ISSN: 1937-335X

## Description

### BACKGROUND OF THE INVENTION

Bone tissue develops and regenerates in adult animals in a metabolic balance between generation by osteoblastic cells and degradation by osteoclastic cells. The regulation of bone regeneration has been extensively studied and is of much current research and clinical interest.

Successful bone growth is required in many different medical treatments in humans such as treatments for congenital anomalities, traumatic injuries to bone, pathological osteolytic conditions and reconstruction of atrophic bone. An area of particular interest is reconstruction of pathologically damaged craniofacial bones and the use of prosthetic devices in dental and orthopedic procedures. Such devices include bone implants for the spine, replacements for major joints as hip and knee, and osseo-integrated dental implants into the jaw. Also, a variety of inflammatory conditions as periodontitis, osteonecrosis, arthritis and osteoporosis are also associated with pathologic osteolysis in craniofacial bone.

In some cases, bone grafting can supply needed bone to supplement surgical procedures by harvesting of autologous bone from sites such as the iliac crest. Currently more than half a million bone grafting procedures are performed in the United States, with the global numbers estimated to be double this figure (Greenwald, et al., 2008) at a cost projected to reach $3.18 billion by 2015. Autologous bone grafting, however, has disadvantages associated with donor site morbidity, infection, nerve injury, pain and significant cost. Young patients have limited donor tissue availability, while the elderly may have poor wound healing from chronic diseases such as diabetes mellitus, osteoporosis or from regimens of bisphophanate drugs, which compromise wound healing. Development of novel cost-effective bone regenerative material could reduce the need for autologous bone grafting for skeletal reconstruction.

In other cases, bone regeneration is not desired and mediation is required to restrain bone growth. Abnormal or ectopic bone formation is a significant clinical problem in diseases such as rheumatoid arthritis, craniosynostosis and fribrodysplasia ossificans progressive, and in formation of bone spurs following bone fracture or bone surgery. In these cases, methods are needed to inhibit the regeneration of bone.

Osteogenesis, the process of bone formation, has been studied extensively. In mammals, it is known that the bone formation occurs by two distinct processes, endochondral (long bone) and intramembraneous (craniofacial bone). The in vitro differentiation of pre-osteoblastic cell lines, such as MC3T3, C2C10, 12 or hFOB and the in vivo differentiation in both of these bone processes have many cellular factors affecting bone processes known to occur in mammals, endochondral (e.g., long bone) and intramembraneous (e.g., craniofacial bone) have intercellular factors that affect bone formation.

Medical implants may be made from various materials, including metals, ceramics, polymers or combinations of these materials. Because these materials can be shaped as needed, prosthetic medical devices are very useful in clinical practice for many different types of surgery involving bone. Osseointegration is the interaction required for medical implants to merge successfully with bone. The physiology of osteogenesis and wound healing in healing of bone is complex. It involves biochemical cascades with specific temporal and spatial requirements for mediators within a local microenvironment.

In medical implant procedures, it is desirable that wound healing occurs rapidly and progresses steadily following the implantation of the medical device to achieve a successful outcome. In this type of surgery, the patient is affected not only by the invasiveness of the surgery itself, but also by the prolonged biological impact and physiological interaction with the implanted device. When a medical implant is present within the wound, as is the case for implant surgery, it is important that the medical implant integrate with the natural process of healing for optimal results.

The physiology of the wound healing with synthetic implants or bone grafting processes is particularly complex. Generally, a sustained sequence of molecular events in the cells and tissue around the surgical site causes the gradual removal and re-modeling of damaged tissue with healthy tissue. These processes involve cascades of biochemical reactions and patterns of timely interactions of multiple factors from the fluids and tissues surrounding the damaged tissue. The timing, order of appearance, and the concentration of multiple biological factors at the wound site are known to affect the rate and outcome of the wound healing process. Biological factors may include growth factors, cytokines, enzymes, hormones and extracellular matrix components.

To improve the wound healing process following implant surgery, one approach is to apply healing compounds to the wound site at the time of surgery. A single application of a bolus of a compound has been provided at the implant site during surgery to reduce inflammation or to provide factors known to be involved in wound healing. For example, a single bolus of growth factor has been placed on or around implants during surgery (Suhonen U.S. Patent 6,132,214; Descouts U.S. Patent 7,090,496). Recently, Clancey et al. (U.S. Patent 7,226,587) disclosed that an application of exogenous recombinant (r) BMP-2 on or around dental and orthopedic implants increased the volume and quality of bone formation around implant devices enhanced osseointegration. Also recently, the FDA has approved use of recombinant human bone morphogenetic protein (rhBMP-2) on collagen membranes for use in implantation in human subjects.

However, the application of a substance at the time of surgery has disadvantages for the wound healing process. Typically, a single bolus of growth factors is placed on or around implants and the timing of the delivery of growth factors cannot be controlled. Because direct access to the site is typically limited to one-time application, i.e., during the surgery, this approach is limited in terms of amount and concentration of the treatment, and therefore the effectiveness is limited and inherently diminishes with time after the surgery. Typically, a single bolus of growth factors is placed on or around implants and the timing of the delivery of growth factors cannot be controlled. Administration of a single bolus of a mediator may not assure that the optimal dose will be available at the required time in the wound healing process. Similarly, recombinant growth factor therapy has a number of disadvantages, including: 1) physiologic doses typically cannot be sustained over extended periods; 2) recombinant growth factors typically are less bioactive than their native counterparts; and 3) recombinant growth factors can be very expensive.

Another approach to enhancing the wound healing process for medical implants is to improve biocompatibility of the medical device by attaching biomolecules to the surface of the medical implant. Examples of attaching biomolecules to medical implant devices are known: Subramaniam (U.S. patent 5,861,032) disclosed a medical device with a biocompatible coating where a bioactive agent is bonded to an organic linker by oxidation. Ellingsen (U.S. patent 7,192,445) disclosed the use of implant devices coated with a layer of biomolecules and titanium hydride. The biomolecules were incorporated into a metal hydride layer as it is formed during electrolysis. Clapper et al. (U.S. patent 6,514,734) disclosed a poly-bi-functional reagent for use as a coating that contains bioactive groups arid latent reactive groups in a polymeric backbone. Clapper et al. (US2003/0181423) further disclosed the use of bone morphogenetic proteins (BMP) and other bone growth factors in a poly-bi-functional reagent. Beyer et al. (U.S. Patent 7,572,766) disclosed peptides for an implant module linked with an analyte module for the binding of BMP.

WO 2008/030611 (D2) describes antibodies to bone morphogenic proteins and receptors therefor and methods for their use.

### SUMMARY OF THE INVENTION

In osseointegration following surgical implantation of a medical device into bone, the bone tissue gradually forms an ordered biological union with the medical implant. (Masuda T, et al., Int J Oral and Maxillofac Implants 1998:13:17-29.). While osseointegration is influenced by many of the molecular and cellular mediators that are normally part of the wound healing process, the members of the Transforming Growth Factor (TGF)-β superfamily play a significant role in mediating the progress of osseointegration. The TGF-β superfamily contains a number of growth factors that share common structural and functional morphology and mediate a variety of biologic functions. The TGF-β superfamily includes. Bone Morphogenetic Proteins (BMP), Activins, Inhibins, Growth and Differentiation Factors (GDF), and Glial-Derived Neurotrophic Factors (GDNF).

The TGF-β BMP family members are considered important growth factor mediators in osteogenesis, especially BMP-2, BMP-4 and BMP-7 (DeBiase P and R Capanna, Injury, 2005, Nov: 36 Suppl 3:543-6; Gautschi et al, ANZ J, Surg. 2007 Aug: 77(8): 626-31). While at least twenty BMP members have been described that share a high level of sequence identity, BMP-2, BMP-4 and BMP-7 stimulate osteoblastic cells leading to new bone formation in in vitro experiments. Agonists and antagonists of BMP are also known. The protein sequences of BMP-2 proteins are known for many species. See, for examples, GenBank accession numbers AAF21646.1 (Homosapiens); CAA8108.1 (Rattes Norvegius) and AAB96785.1 (Oryctolagus cuniculus),

Novel therapeutic modalities for bone regeneration involve immobilization of anti-BMP-2 antibody on a solid scaffold. That is used to capture endogenous BMP-2. This modality is termed antibody-mediated-osseous regeneration (AMOR). In order to participate in AMOR, an antibody (Ab) molecule must possess the following properties: 1) ability to bind endogenous BMP-2 with high affinity; 2) ability to bind to a BMP-2 epitope remotely from the BMP-2 receptor-binding domains, 3) ability to form an Ab-BMP-2 immune complex capable of binding the BMP-2 cellular receptor on osteoprogenitor cells; 4) ability to form an Ab-BMP-2 immune complex that is able to transduce intracellular signals 5) ability to form an Ab-BMP-2 immune complex that mediates osteogenic differentiation, and 6) avoidance of adverse local or systemic immunological response in the host.

Experimental results identified monoclonal antibodies (MAbs) that specifically bound BMP-2, allowed BMP-2 to bind to osteogenic cells, and enhanced bone regeneration. Other MAbs were identified that prevented the bound BMP-2 and in turn the MAB-BMP-2 complex bound to BMP-2 receptors on cells, as well as MABs that bound BMP-2 while preventing binding of BMP-2 to its receptor and limited osteogenic differentiation. An affinity purified polyclonal rabbit anti-human BMP-2 was also found to be able to bind BMP-2 and to allow the BMP to bind to target cells. The polyclonal Ab is likely to contain antibodies which react with a multitude of epitopes, some of which may block binding of BMP-2 in the immune complex to its cellular receptor, while other epitopes do not interfere with this binding.

One of the requirements for AMOR is that antibodies should not generate an adverse local or systemic immunological response in the host. In all of the histological sections examined following AMOR in rat and rabbit calvarial defects, no significant evidence of inflammatory infiltration has been noted. Moreover, necropsy examination of rats and rabbits did not reveal any systemic evidence of pathology.

Antibodies were screened and selected for properties that mediated bone regeneration. A select group of antibody clones (4B12, 3G7 and C22) had properties that enhanced osteogenesis and showed significant increase in bone regeneration as compared with both isotype controls and negative controls. These antibodies will be useful in those diseases or procedures requiring bone regeneration. Another group of antibodies (such as C13) that inhibited bone regeneration may have application in those instances where bone regeneration should be decreased.

Selected antibodies captured BMP-2 as it was normally expressed in the vicinity of osteogenic cells during wound healing and localized BMP-2 to the implant area and improved the wound healing process. When the naturally occurring antigenic factor normally appears during wound healing, the selected antibodies captured the antigenic factor onto the surface of the implant, thus increasing the concentration of the selected factor, as well as extending the length of time of exposure of the implanted device to the antigenic factor. This modification of the surface of the medical implant by binding of specific antibody enhanced the bioavailability of native growth factors and improved wound healing.

For the selected bone morphogenetic protein (BMP) antibodies, other embodiments include polyclonal, monoclonal or recombinant/synthetically generated immmunoglobulins, as well as antibody fragments with antigen binding function: Fab, F(ab')₂, single-chain variable region antibody fragment (scFv), minibody, and complementary determining region (CDR) are utilized. Antibodies, antibody fragments or mixtures of antibodies may be used.

The BMP-2 growth factor-antibody immune complex at the site of the implant enhanced the osteogenic process at the site and ultimately the osseo-integration of dental and orthopedic implants to which it was attached. The density of bone around the implant was increased as determined both by micro-computed tomorgraphic imaging and histological observations.

This procedure allows for the capture and concentration of native biomolecules on an implanted medical device. The coupling of growth factor release to the normal physiologic process of osteogenesis is driven by in vivo growth factor production. The capture and concentration of native biomolecules are more likely to be timely produced and to be more bioactive than their recombinant counterparts.

This application claims priority to U.S. Provisional Application Serial No. 61/145,963 filed January 20,2009 and to U.S Provisional Application Serial No. 61/172,666 filed April 24, 2009.

### BRIEF DESCRIPTION OF THE FIGURES

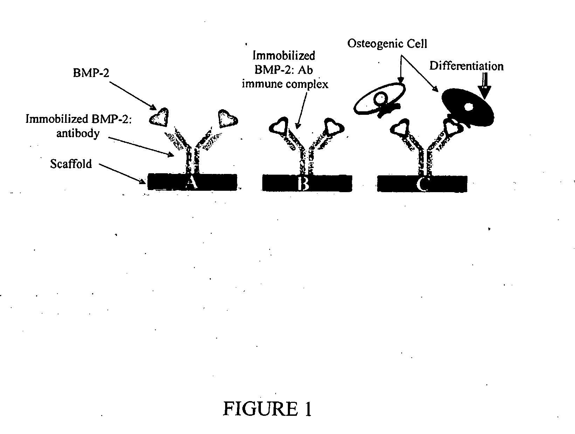

FIGURE 1 shows strategy for antibody mediated osseous regeneration (AMOR). (A) Anti-BMP-2 antibodies are immobilized on a carrier or scaffold; (B) Antibodies capture endogenous BMP-2 from the microenvironment, concentrating BMP-2 near the bone regeneration site; (C) BMP-2 captured by specific antibody binds its cellular receptor on osteoprogenitor cells, promotoing their osteogenic differentiation.

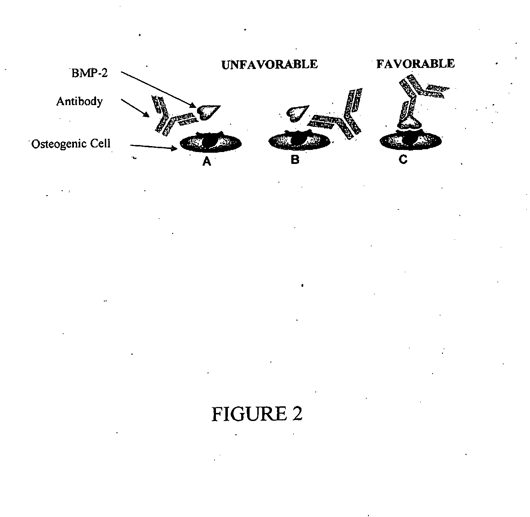

FIGURE 2 shows strategy of consequences of antibody binding to various BMP-2 epitopes. (A) Antibody binding to BMP receptor-specific domains (wrist and knuckle) prevented BMP-receptor interactions: (B) Antibody binding to epitopes near receptor-specific domains created steric hindrance; (C) Antibody binding to epitopes remote from receptor-specific domains can mediate antibody mediated osseous regeneration (AMOR) because it does not interfere with BMP-receptor interactions.

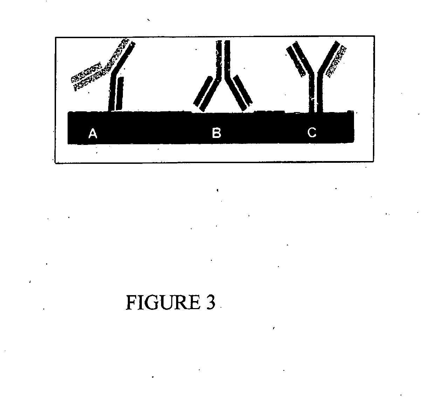

FIGURE 3 shows strategy of the influence of antibody orientation following immobilization on carrier or scaffold on the availability of antigen-binding sites. (A) Availability of one complementary determining region (CDR); which may be potentially spacially unavailable for simultaneously interacting with BMP and its cellular receptor; (B) No CDR available for binding; and (C) Both CDR's available for binding with antigen.

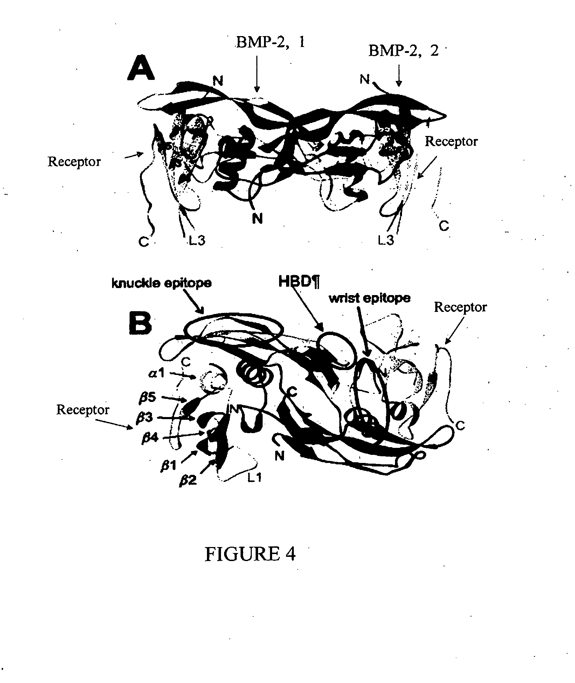

FIGURE 4 shows the tertiary structure of the BMP-2-BRIA ectodomain complex. A ribbon representation showing (A) the side view with the membrane proximal side on the bottom and (B) top view along the twofold axis of the complex. The BMP monomers are medium gray and darker gray, while the two BRIA ectomain molecules are light gray. The "wrist" and "knuckle" epitopes, as well as the heparin binding domain (HBD) located at the N-terminal of BMP-2 are indicated (adapted from Kirsch et al., 2000).

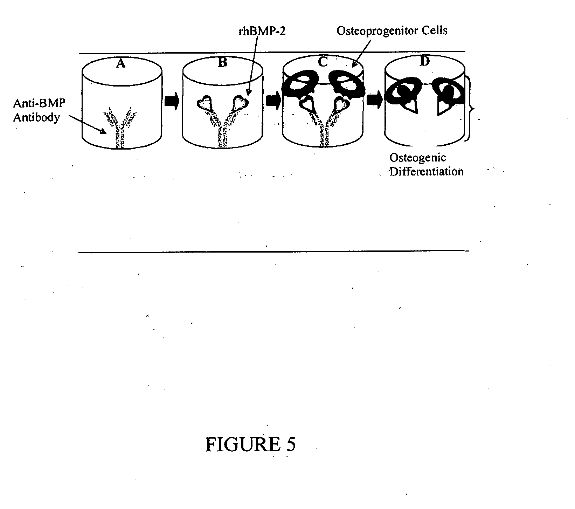

FIGURE 5 shows the experimental strategy to (A) immobilize anti-BMP antibodies on culture plates; (B) incubate with low concentration rhBMP-2 (10 ng/ml); (C) incubate with C2C12 osteoprogenitor cells and (D) assay for osteogenic differentiation of cells by alkaline phosphatase reaction (ALP).

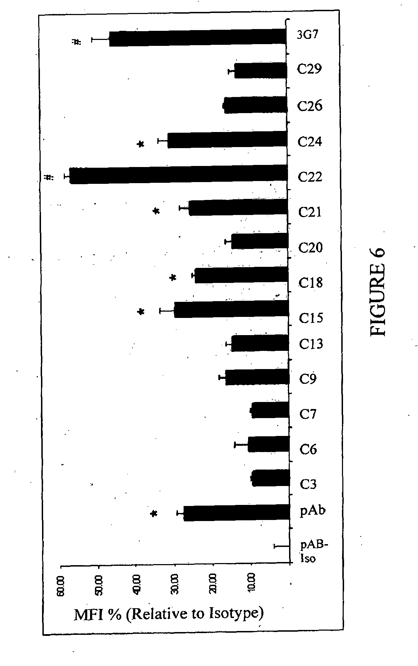

FIGURE 6 shows flow cytometric analysis of binding of simultaneous binding of various anti-BMP-2 antibody clones to rhBMP-2 immune complexes and to C2C12 osteogenic cells. The MFI percentage relative to isotype is indicated for control of isotype matched (Ablso), polyclonal antibody (pAb), 3G7S clone, and various monoclonal clones obtained from a mouse hybridoma library (C3, C6, C7, C9, C13, C15, C18, C20, C21, C22, C24, C26, and C29). Significance of binding at p<0.05 (*) and p< 0.01 (#) is indicated.

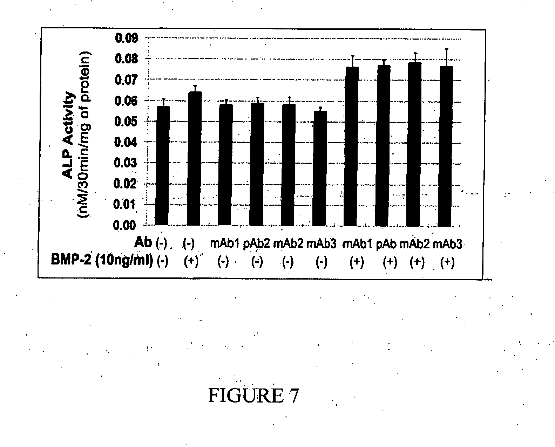

FIGURE 7 shows alkaline phosphatase activity of hFOB cell lines cultured in the presence (+) or absence (-) of BMP-2 and antibody clones. Controls: no antibody, no BMP-2, and BMP-2 without antibody. Antibody clones were tested without added BMP-2 (-), and then with added BMP-2 (+) to form immune complexes. Samples tested: mAb1; pAb2, mAb2 and mAb3. Ab1 (clone 100221, R&D Systems: www.rndsystems.com); pAb (affinity purified goat anti-BMP-2 pAb (R&D Systems; www:rndsystems.com); mAb2 (clone 100230, R&D Systems; www.mdsystems.com) and mAb3 (clone 65529, R&D Systems; www.mdsystems.com) were tested.

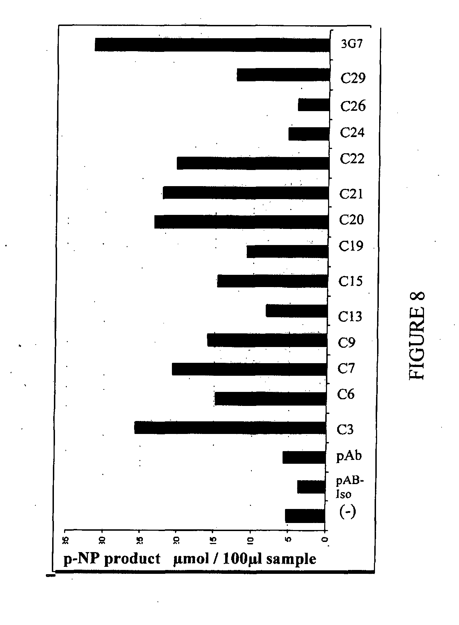

FIGURE 8 shows alkaline phosphatase activity of C2C12 cell lines cultured for two days in the presence or absence of rhBMP-2 and various antibody immune complexes.

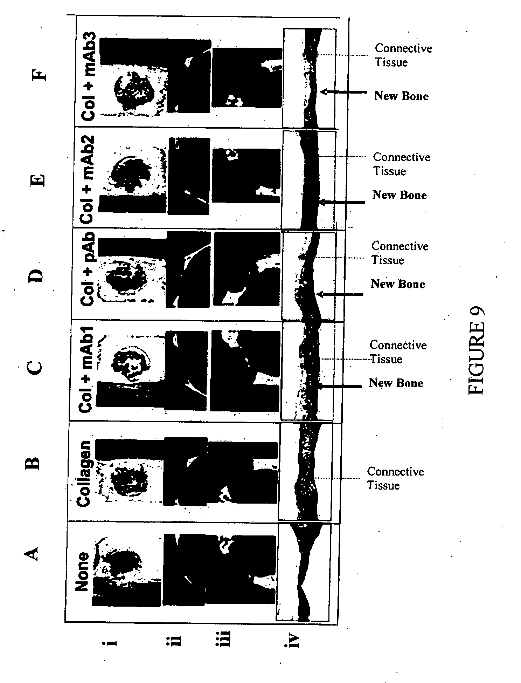

FIGURE 9 shows bone regeneration obtained in rat calvaria defects two weeks after surgery. Four mm surgical defects were created, filled with controls or BMP-2 specific antibodies immobilized on collagen and tested after two weeks of healing. FIGURE 9A: sample panels 1-6 contain views of micro-CT scans of (i) cross section of defect area; (ii) coronal section of defect, (iii) detail of coronal section. Radio lucency (dark area) indicates a vacancy. Radio opacity (light areas) indicates dense tissue. Section (iv) shows corresponding histology of a representative region stained by hemotoxylin and eosin. The dotted arrows indicate connective tissue, solid arrows indicate new bone. Samples: (A) controls: defect unfilled (none) and (B) defect filled with isotypic antibody immobilized on collagen. Experimentals: defect filled with collagen + BMP-2 binding monoclonal antibody: (C) monoclonal antibody mAb1 (clone 100221; www.rndsystems.com); (D) polyclonal antibody pAb (affinity purified goat anti-BMP-2 pAb; R&D Systems; www.rndsystems.com); (E) monoclonal antibody mAb2 (clone 100230, R&D Systems; www.rndsystems.com), and (F) monoclonal antibody mAb3 (clone 655529, R&D Systems; www.rndsystemscom).

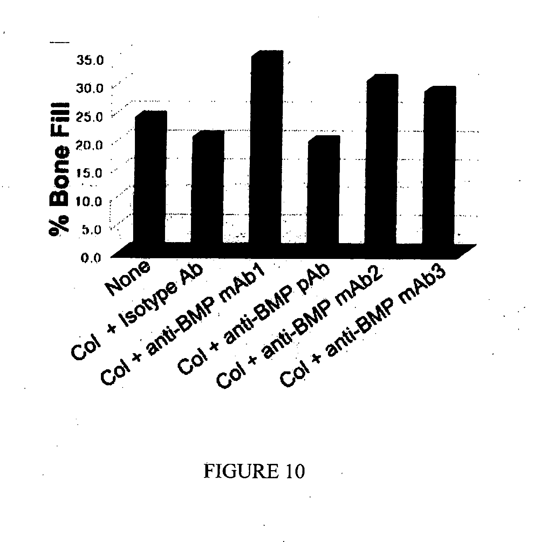

FIGURE 10 shows histomorphometry indicating the % bone fill in each defect area for each sample tested in FIGURE 9.

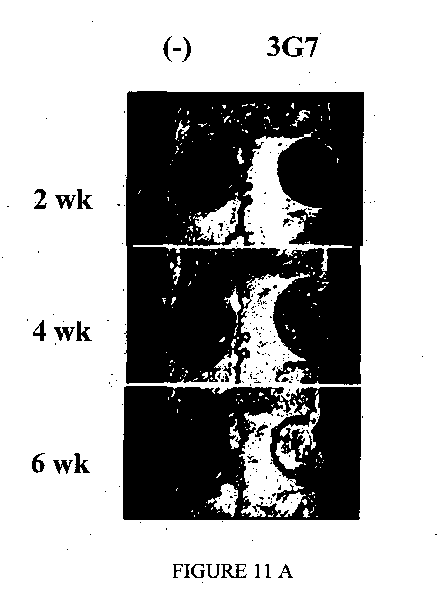

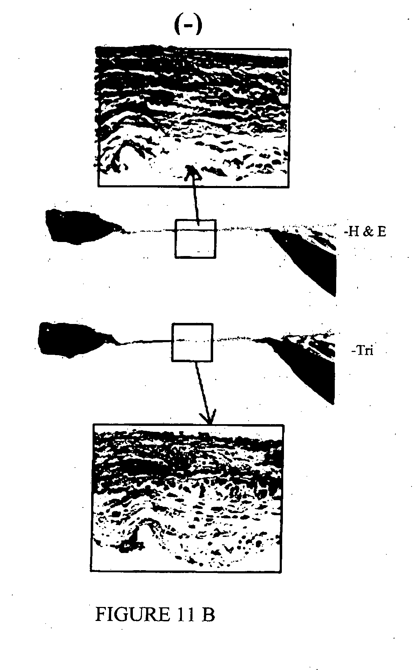

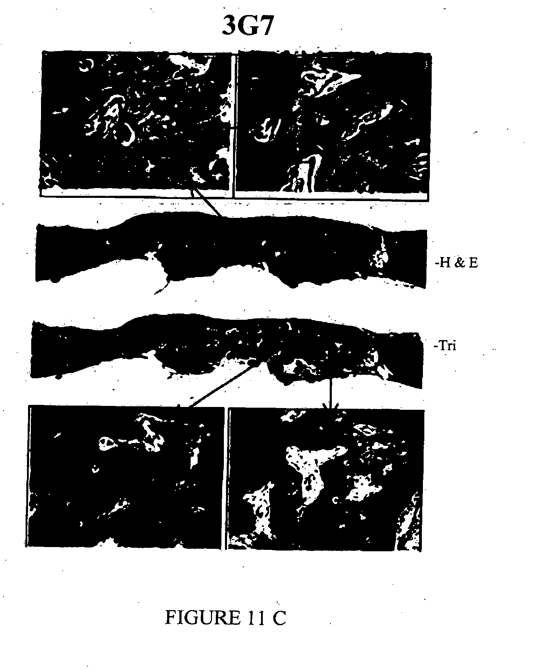

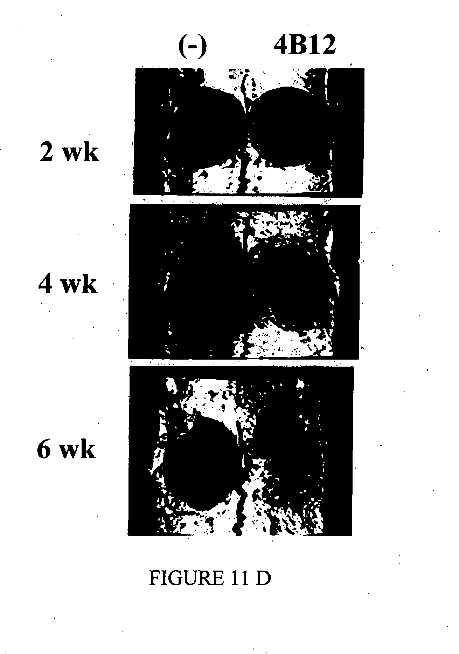

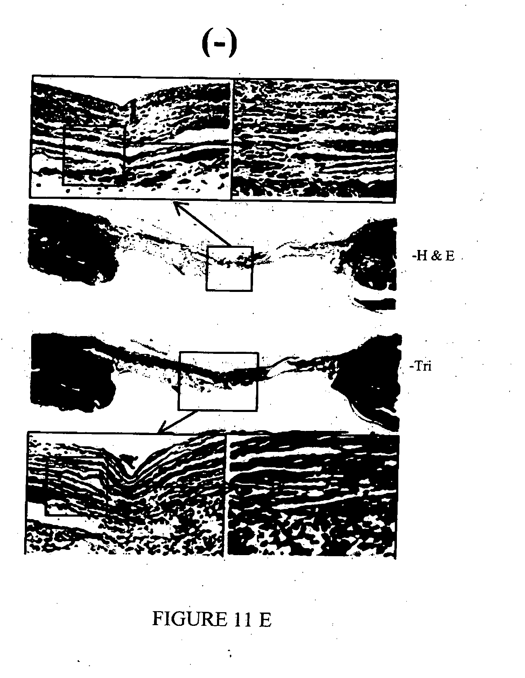

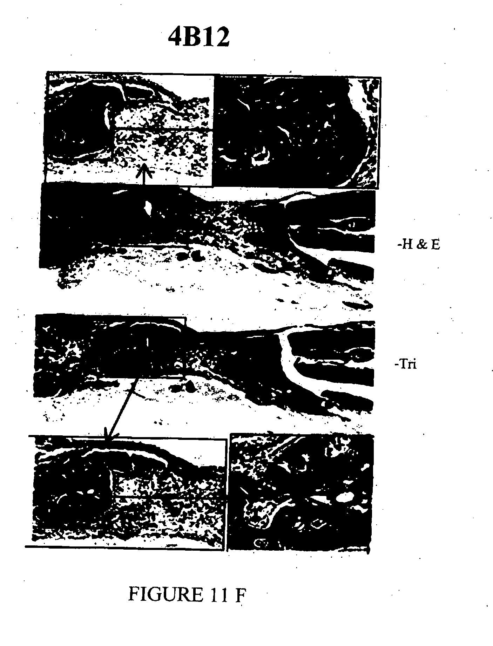

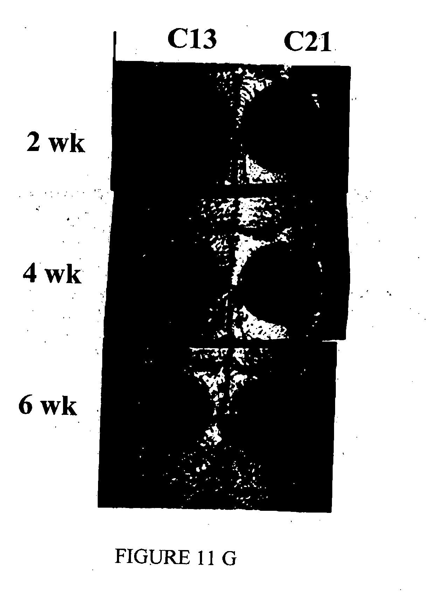

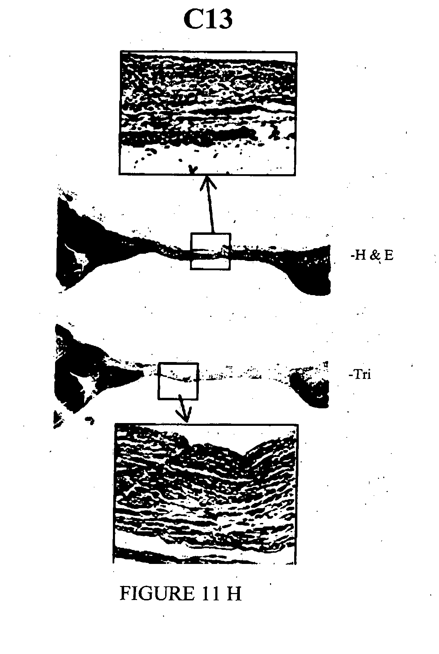

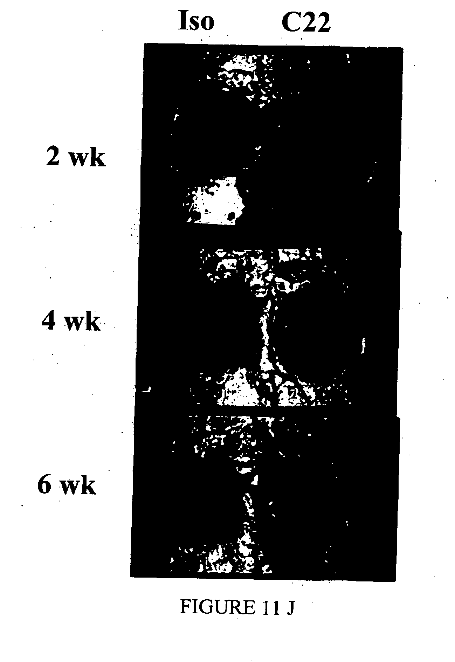

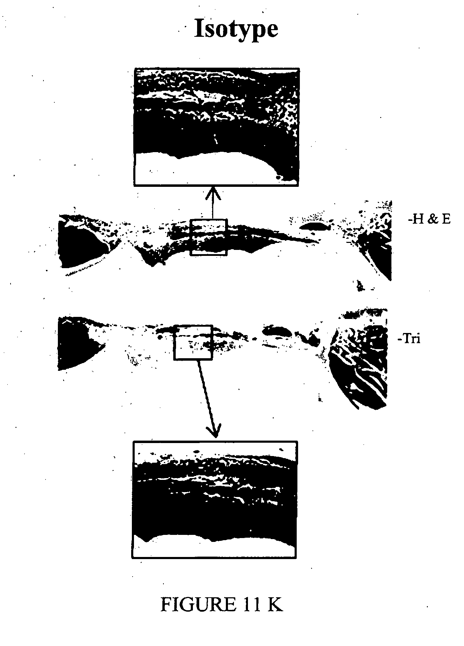

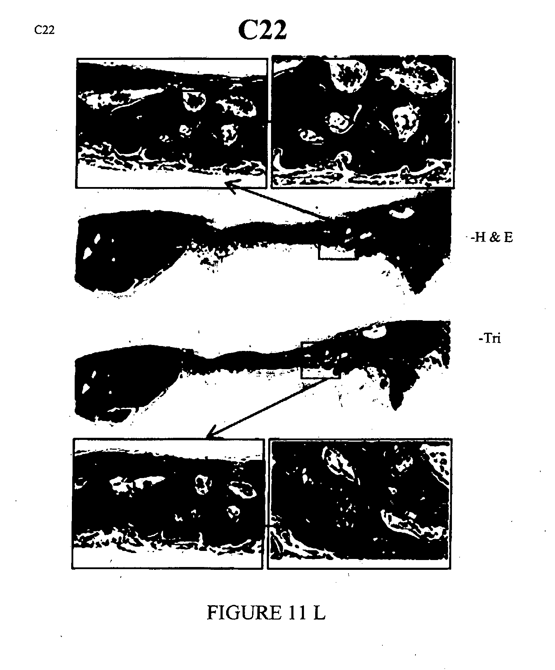

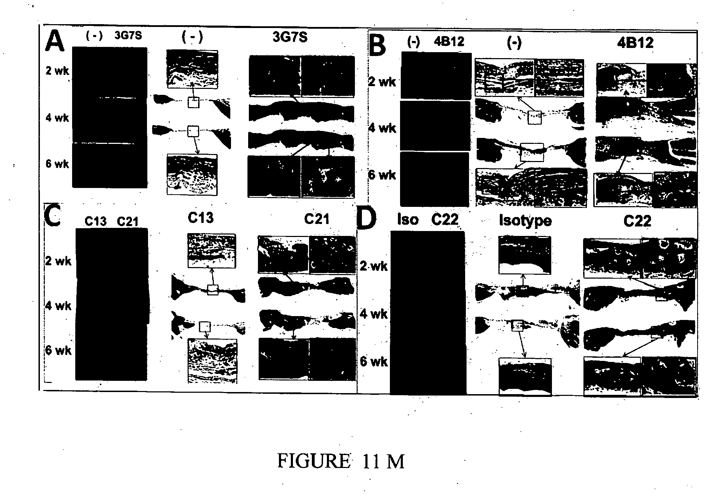

FIGURES 11A -11M shows antibody mediated osseous regeneration (AMOR) within rat calvarial defects treated with controls or immobilized experimental monoclonal antibody clones. Defect regions were examined by µ-CT cross-sections at two, four and six weeks after surgery, and by histological examination (hematoxylin and eosin; trichrome stain) and histomorphometry measurement following euthanasia of animals at six weeks. Detail enlargements are indicated by boxed area and arrow. Results were representative of five experiments. FIGURE 11A: Comparison of µ-CT for control (-) (collagen membrane only) and clone 3G7; FIGURE 11B: Histology of control (-) at six weeks; FIGURE 11C: Histology of clone 3G7; FIGURE 11D: Comparison of µ-CT for control (-) and clone 4B12; FIGURE 11E: Histology of control (-) at six weeks; FIGURE 11F: Histology of clone 4B12; FIGURE 11G: Comparison of µ-CT for clones C13 and C21; FIGURE 11H: Histology of clone C13; FIGURE III: Histology of clone C21; FIGURE 11J: Comparison of µ-CT for isotype match control and clone C22; FIGURE 11K: Histology of isotype control; and FIGURE 11L: Histology of clone C22. For comparison, FIGURE 11M presents FIGURES 11A-11L aligned on one page.

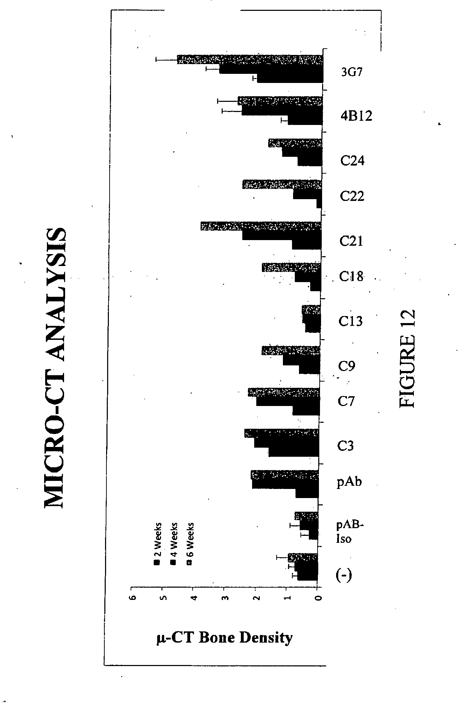

Figure 12 shows a bar graph of the µ-CT bone density obtained at the three time points of two weeks, four weeks and six weeks for controls (-) and isotype matched antibody (iso). Experimentals shown are polyclonal antibody clone (pAB1), monoclonal antibody clones 4B12 and 3G7S, as well as monoclonal antibodies from a created library (C9, C18, C13, C3, C24, C7, C22, C21).

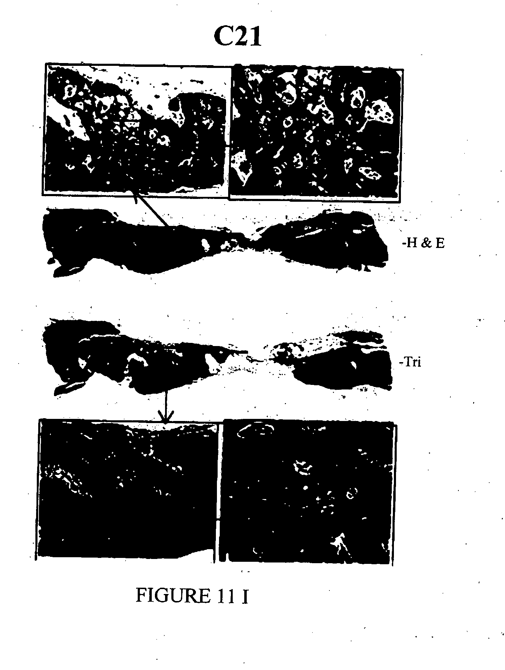

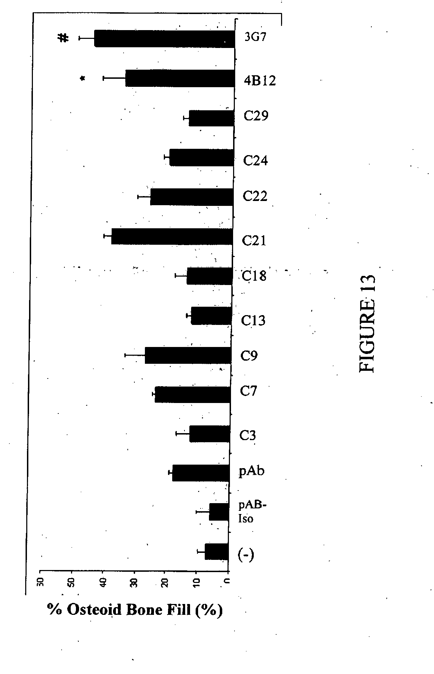

FIGURE 13: Histomorphometry measurement of osteoid bone fill at six weeks after surgery for samples in Figure 11 and Figure 12. The percentage of osteoid bone fill is indicated. Two-tailed T test demonstrated significance at p< 0.05 (*) for clone 4B17 [4B12?] and p<0.01 (#) for clone 3G7S.

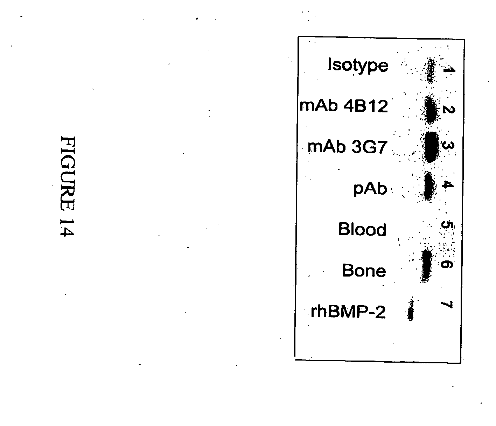

FIGURE 14 shows a Western blot analysis of proteins eluted from immunomagnetic beads. Lanes are: (1) isotype antibody; (2) monoclonal antibody clone 4B12; (3) monoclonal antibody 3G7S; (4) polyclonal antibody clone pAb; (5) blood homogenate; (6) bone homogenate; and (7) 2 ng of rhBMP-2.

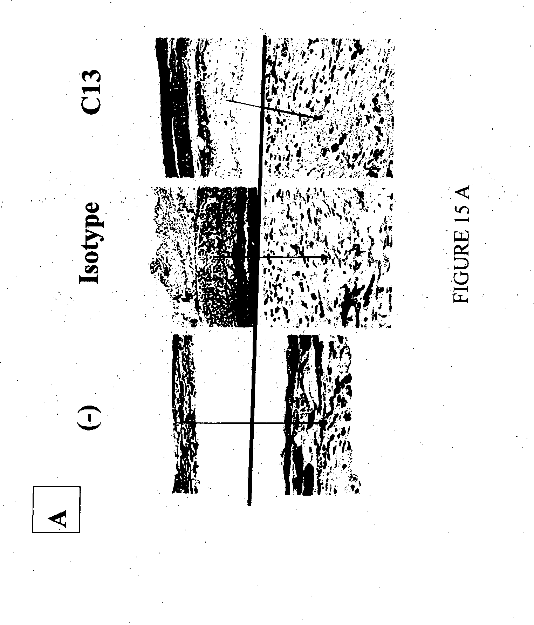

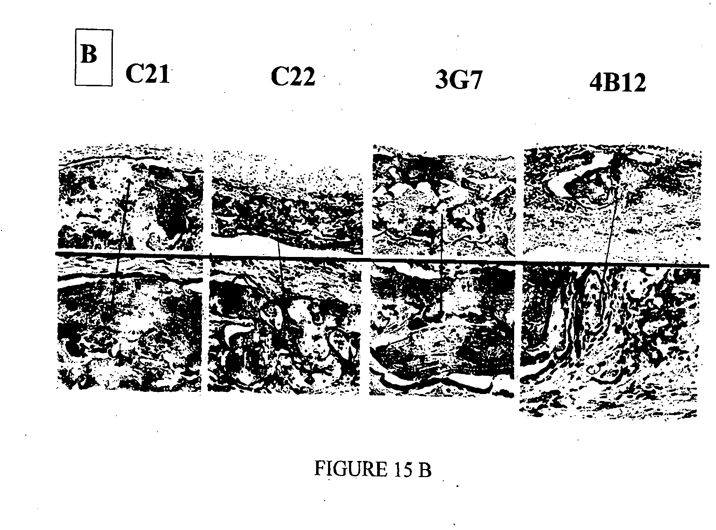

FIGURES 15A -15B show in situ distribution of expression of BMP-2 (A-B) and osteocalcin (C -D) by immunohistochemistry. Arrow indicates detail of a section. Sections were labeled with anti-BMP-2 polyclonal antibodies followed by HRP-conjugated secondary antibody binding. FIGURE 15A shows BMP-2 distribution for controls of membrane alone (-) and isotype matched antibody (isotype), and monoclonal antibody clone C13; FIGURE 15B shows monoclonal antibody clones C21, C22, 3G7 and 4B12.

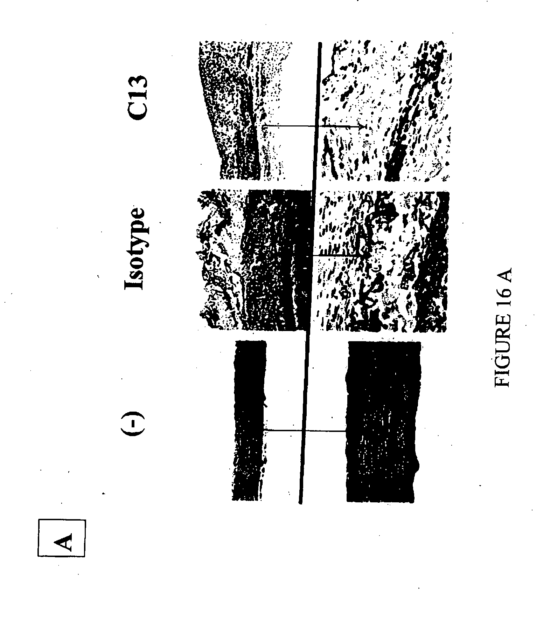

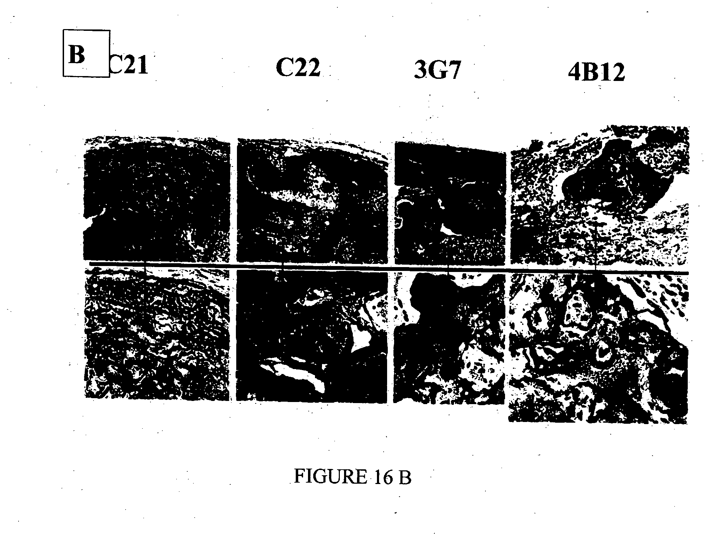

FIGURES 16A - 16B show in situ distribution of osteocalcin by immunohistochemistry. FIGURE 16A shows osteocalcin distribution in controls (-) and isotype-matched antibody control (Iso), and for monoclonal antibody clone C13; FIGURE 16B shows osteocalcin distribution in monoclonal antibody clones C21, C22, 3G7 and 4B12.

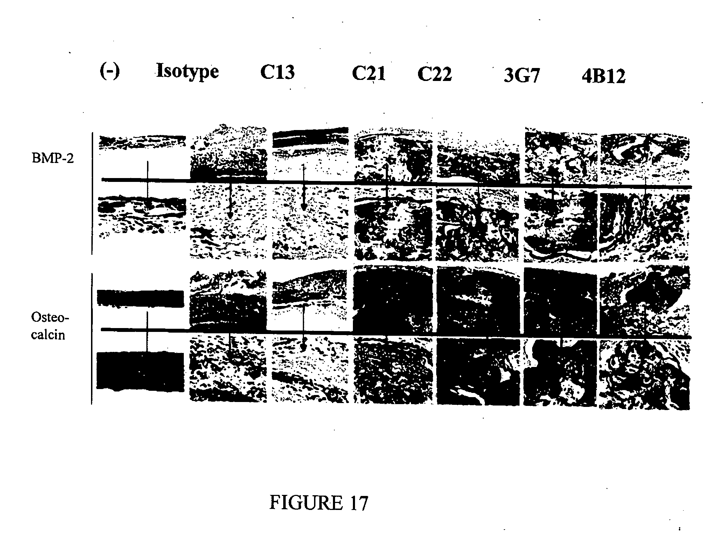

FIGURE 17 shows FIGURES 15 and Figure 16 aligned on one page for comparison of expression of BMP-2 and osteocalcin.

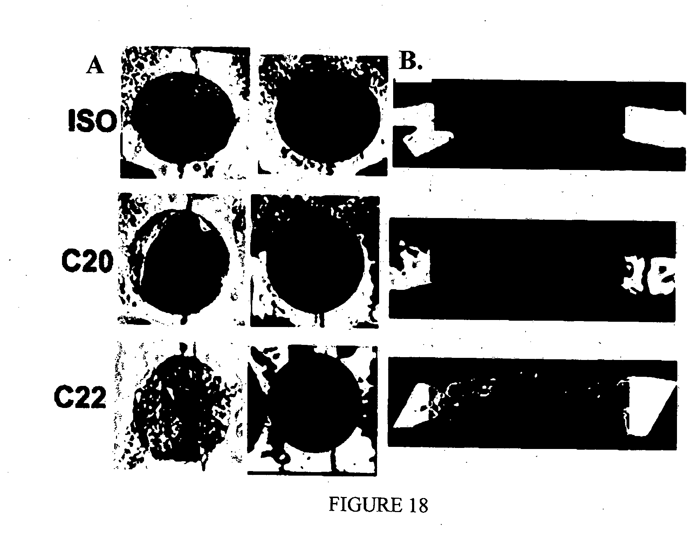

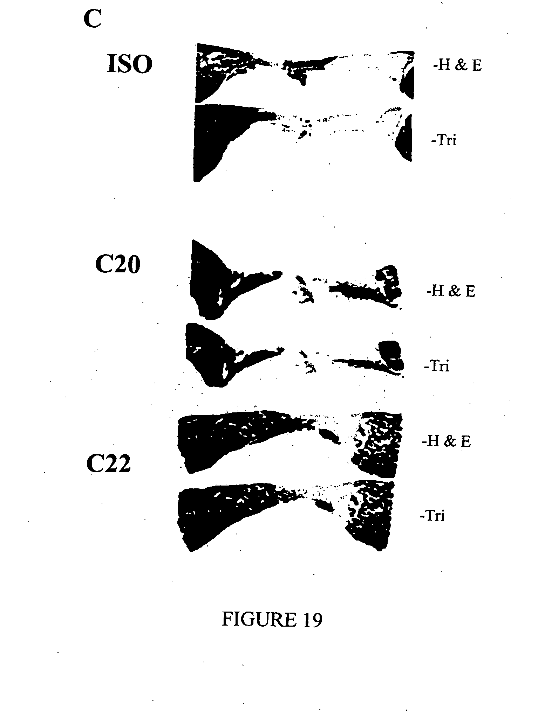

FIGURES 18A -18B show antibody mediated osseous regeneration (AMOR) within rabbit calvarial defects at four weeks after surgery. FIGURE 18A shows µ-CT results for calvarial defect cross sections; FIGURE 18B shows µ-CT results of coronal sections of the treated defect surgical area. Antibodies used in FIGURES 18 A - B and Figure 19 included isotype matched antibody (Iso), monoclonal antibody clones C20 and C22.
FIGURE 19 shows histological analysis for both hemotoxylin and eosin staining (H & E) and trichrome (tri) at 4 weeks for the samples in FIGURE 18.

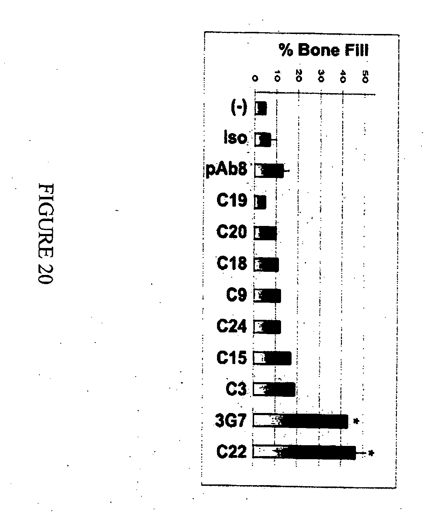

FIGURE 20 shows histomorphological measurement of the samples in Figures 18 and 19 for the antibody clones Iso, C20 and C22, as well as other antibodies tested in rabbit calvarial defects, including anti-BMP-2 polyclonal.antibody (pAb8), or various anti-BMP-2 monoclonal antibody clones (C19, 18, C9, C24, C15, C3, 3G7S). T-test relative to isotype-matched-antibody, p< 0.05 (*) is indicated.

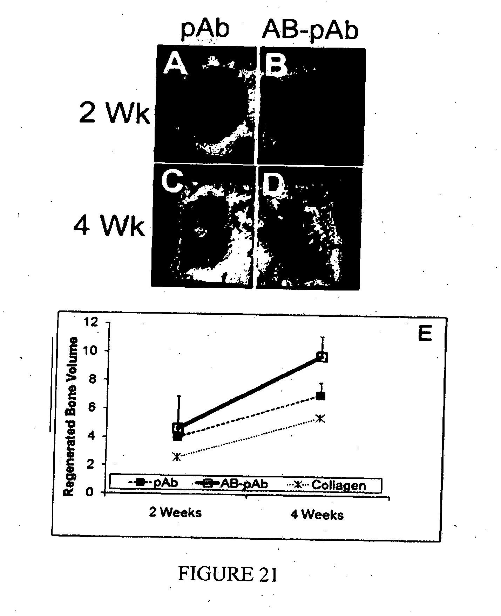

FIGURE 21A - D shows µ-CT scans of cross sections of rat calvarial defects at two weeks (A-B) and four weeks (C-D) for polyclonal antibody clone pAb and for the heparin-absorbed pAb antibody (AB-pAb). Figure 21E shows the histomorphometric quantitation of the regenerated bone volume for collagen control (stars), pAb (closed squares) and heparin-absorbed pAb antibody (open squares).

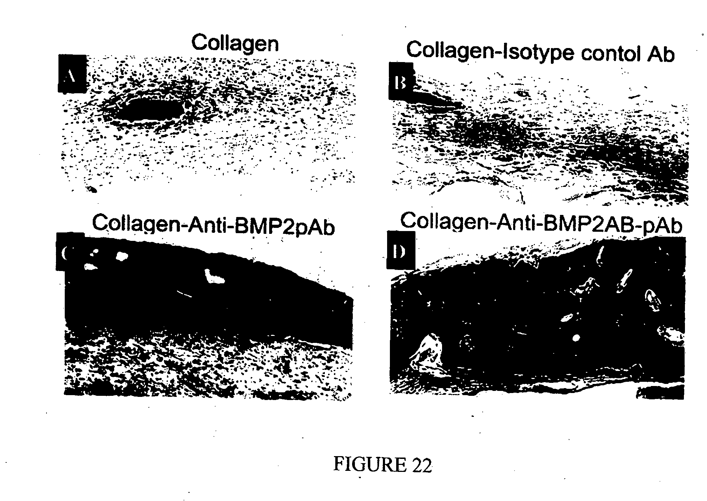

FIGURES 22 A -D shows histological comparisons obtained four weeks after surgery when rat calvarial defects shown in Figure 21 were filled with (A) collagen alone; (B) collagen plus isotype control antibody; (C) collagen plus antiBMP-2 polyclonal antibody clone pAb; and (D) collagen plus anti-BMP-2 pAb absorbed on a heparin-BMP-2 column (AB-pAb).

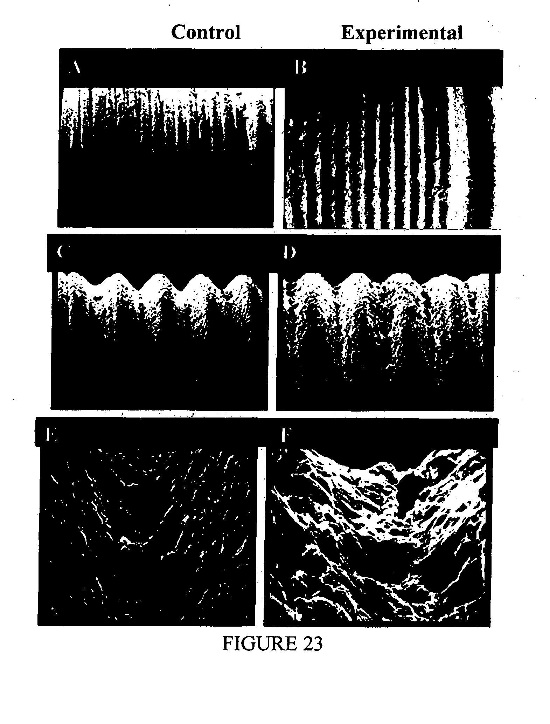

FIGURES 23 A-F show scanning electron microscopy (SEM) of the in vivo cell attachment to Astratech osseospeed implants (3.5 X 8 mm) coated either with isotype control (A, C, E) or with experimental BMP-2-specific antibody clone (B, D, F). Samples were harvested after 7 days of placement into rabbit tibia. FIGURES 23 A - B show the microthread portion. FIGURES 23 C - D show macrothread portions of the implants, and FIGURES 23 E - F are high magnification views of these macrothreads. FIG. A (30x magnification); FIG. B (38x magnification); FIGS. C and D (40x magnification); FIGS. E and F (500x magnification).

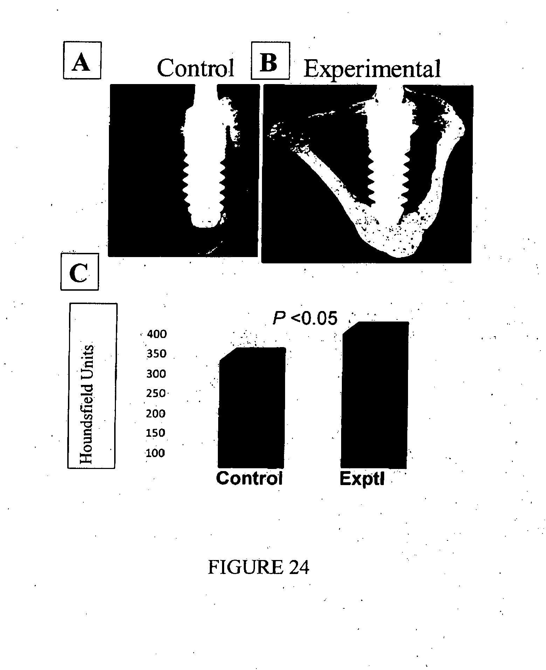

FIGURES 24 A-C show µ-CT data on the BMP-2 specific antibody coated and isotype control antibody coated implants harvested after 14 days placement of Astratech osseospeed implants (3.5 x 8 mm) into rabbit tibia. FIGURE 24 A shows representative images of the µ-CT image of control implant, while FIGURE 24 B shows the µ-CT image of experimental BMP-2 antibody clone mAb1 (clone 100221, R&D Systems; www.rndsystems.com). FIGURE 24 C shows the quantitative measurement of density of bone around the experimental and control implants shown in FIGURES 24 A -B. Units are expressed in Houndsfield units. T-test showed p<0.05 for experimental as compared with control.

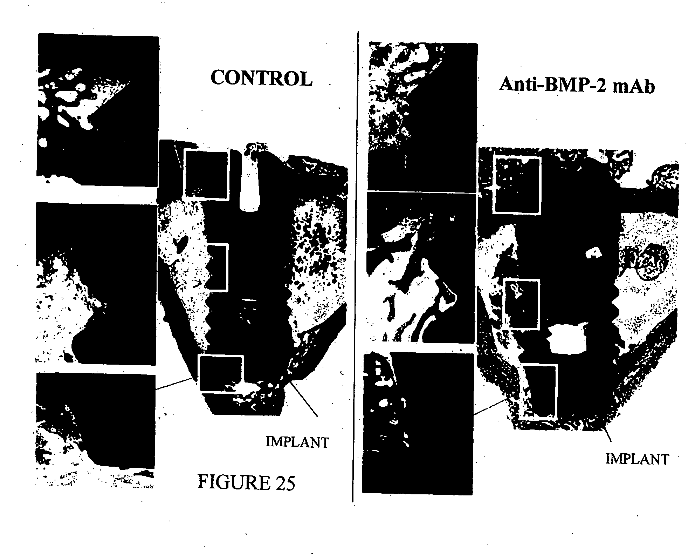

FIGURES 25 A-H show histological sections of immunoglobulin isotype-matched control (A - D) and experimental BMP-2-specific antibody (mAb1, clone 100221) coated implants (E - H). Implants were placed into rabbit tibia and harvested 18 days after surgery.

### DETAILED DESCRIPTION OF THE INVENTION

The biologic performance of a variety of implantable devices can be improved by capturing biomolecules in vivo using immobilized antigen-binding molecules. The devices include dental implants, dental implant prosthetic components (eg. abutments), orthopedic implants, central lines, catheters, implantable drug delivery devices, and pacemakers. The material structure of such devices to be modified may include titanium and its alloys, zirconium and its alloys, vanadium and its alloys, caladium and its alloys, gold and its alloys, aluminum oxide, stainless steel, as well as other ceramics, plastics, resins and metals.

Figure 1 shows the basic strategy devised and tested for antibody mediated osseous regeneration (AMOR). In Figure 1A, anti-BMP-2 antibodies are immobilized on a scaffold which is inserted in an in vivo regeneration site for bone tissue. The immobilized antibodies capture endogenous BMP-2 from the microenvironment, effectively concentrating BMP-1 near the regeneration site (Figure 1B). BMP-2 capture,by BMP-2 specific antibody binds cellular receptors on osteoprogenitor cells, promoting osteogenic differentiation (Figure 1C). In AMOR, immobilized antibodies on a scaffold specifically concentrated BMP-2 antigen at the site of the scaffolding. Receptors on the cell surface of osteogenic cells bound the BMP-2: antibody immune complex, inducing cell signaling for osteogenic differentiation and allowing enhancement of bone regeneration in the region surrounding the scaffold. The capture of endogenous BMP-2 increased the concentration and availability of BMP-2 factor at the regeneration site.

Various BMP-2 specific antibodies bind different epitopes on the BMP-2 protein. Figure 2A-C shows consequences of antibody binding to various BMP-2 epitopes. Binding of antibody to the BMP receptor specific domains ("wrist" and "knuckle") could prevent proper BMP-receptor interactions (Figure 2A). Another unfavorable binding is to epitopes near the receptor -specific domains that could create steric hindrance (Figure 2B). Preferred antibody binding was to epitopes on the BMP-2 molecule that are separate from receptor-specific domains to avoid interference with BMP-receptor interactions (Figure 2C).

Another strategy was the immobilization of the antibody to the carrier or scaffold could affect the binding ability of the immobilized antibody. This strategy is shown in Figure 3A-C. Unfavorable binding scenarios are shown in Figures 3A and 3B. Theoretically, Figure 3C would be a preferred orientation of the immobilized antibody for binding of antigen.

Yet another strategy was binding access to other epitopes on the BMP-2 protein may be important to distinguish effects of various antibody clones on bone regeneration. In addition to avoiding hindrance of the receptor binding domains (wrist and knuckle), availability of the heparin-binding domain (HBD) for antibody binding was considered (Figure 4A-4B). Figure 4A shows tertiary structure in ribbon representation of the complex between the dimer of BMP-2 bound to two BRIA receptor ectodomains. By rotating this view slightly to the right, the heparin-binding domain is indicated in Figure 4B (figure adopted from Kirsch et al., 2000). In this manner, other epitopes may be present that influence interaction with BMP-2 agonists and antagonists and determine ultimate effect on bone regeneration.

Figures 5A - D show the strategy devised for in vitro assay of osteogenic differentiation for immobilized BMP-2 specific antibodies. This approach was used for flow cytometric analysis and for alkaline phosphatase assay. Antibodies to be tested were immobilized on culture plates (Figure 5A). The plates were incubated with a concentration of rhBMP-2 that by itself had no osteogenic effect (10 ng/ml) (Figure 5B) and washed. Osteogenic cells, such as mouse myoblast cell line C2C12, mouse osteoblast cell line MC3T3-E1, or human osteoblast cell line hFOB1.19, were then added to the culture plates (Figure 5C). After a time interval, the amount of osteogenic differentiation was determined by flow cytometry or alkaline phosphatase reactivity.

In the Examples provided below, monoclonal antibodies were produced using established protocols. See, e.g., (Galfre G and Milstein C, 1981, Methods Enzmol., 73 (Pt B): 3-46; Milstein C, 2003, Immunol. Today, Aug 21 (8): 359-64).

The antibodies described herein include derivatives that are modified, i. e, by the covalent attachment of any type of molecule to the antibody such that covalent attachment does not prevent the antibody from generating an anti-idiotypic response. For example, but not by way of limitation, the antibody derivatives include antibodies that have been modified, e.g., by glycosylation, acetylation, pegylation, phosphylation, amidation, derivatization by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, metabolic synthesis of tunicamycin, etc. Additionally, the derivative may contain one or more non-classical amino acids.

Polyclonal antibodies to BMP-2 can be produced by various procedures well known in the art. For example, a polypeptide of the invention can be administered to various host animals including, but not limited to, rabbits, mice, rats, etc. to induce the production of sera containing polyclonal antibodies specific for the antigen. Various adjuvants may be used to increase the Immunological response, depending on the host species, and include but are not limited to, Freund's (complete and incomplete), mineral gels such as aluminum hydroxide, surface active substances such as lysolecithin, pluronic polyols, polyanions, peptides, CpG or other modified oligonucleotides, oil emulsions, keyhole limpet hemocyanins, dinitrophenol, and potentially useful human adjuvants such as BCG (bacille Calmette-Guerin) and corynebacterium parvum. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies A Laboratory Manual, (Cold Spring Harbor Laboratory Press, 2nd ed. 1988); Hammerling, et al., in: Monoclonal Antibodies and T-Cell Hybridomas (Elsevier, N.Y., 1981). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced,

Methods for producing and screening for specific antibodies using hybridoma technology are routine and well-known in the art. Briefly, mice can be immunized with a polypeptide of the invention or a cell expressing such peptide. Once an Immune response is detected, e.g., antibodies specific for the antigen are detected in the mouse serum, the mouse spleen is harvested and splenocytes isolated. The splenocytes are then fused by well-known techniques to any suitable myeloma cells, for example cells from cell line SP20 available from the ATCC. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Another well known method for producing both polyclonal and monoclonal human B cell lines is transformation using Epstein Barr Virus (EBV). Protocols for generating EBV-transformed B cell lines are commonly known in the art, such as, for example, the protocol outlined in Chapter 7.22 of Current Protocols in Immunology, Coligan et al., Eds., 1994, John Wiley & Sons, NY. The source of B cells for transformation is commonly human peripheral blood, but B cells for transformation may also be derived from other sources including, but not limited to, lymph nodes, tonsil, spleen, tumor tissue, and infected tissues. Tissues are generally made into single cell suspensions prior to EBV transformation. Additionally, steps may be taken to either physically remove or inactivate T cells (e.g., by treatment with cyclosporin A) in B cell-containing samples, because T cells from individuals seropositive for anti-EBV antibodies can suppress B cell immortalization by EBV. In general, the sample containing human B cells is innoculated with EBV, and cultured for 3-4 weeks. A typical source of EBV is the culture supernatant of the B95-8 cell line (ATCC #VR-1492). Physical signs of EBV transformation can generally be seen towards the end of the 3-4 week culture period. By phase-contrast microscopy, transformed cells may appear large, clear, hairy and tend to aggregate in tight clusters of cells. Initially, EBV lines are generally polyclonal. However, over prolonged periods of cell cultures, EBV lines may become monoclonal or polyclonal as a result of the selective outgrowth of particular B cell clones, Alternatively, polyclonal EBV transformed lines may be subcloned (e.g., by limiting dilution culture) or fused with a suitable fusion partner and plated at limiting dilution to obtain monoclonal B cell lines. Suitable fusion partners for EBV transformed cell lines include mouse myeloma cell lines (e.g., SP2/0, X63-Ag8.653), heteromyeloma cell lines (human x mouse; e. g, SPAM-8, SBC-H20, and CB-F7), and human cell lines (e.g., GM 1500, SKO-007, RPMI 8226, and KR-4).

Accordingly, the present disclosure includes methods of generating monoclonal antibodies as well as antibodies produced by the method comprising culturing a hybridoma cell secreting an antibody of the invention wherein, preferably, the hybridoma Is generated by fusing splenocytes isolated from a mouse immunized with an antigen of the invention with myeloma cells and then screening the hybridomas resulting from the fusion for hybridoma clones that secrete an antibody able to bind a polypeptide of the invention.

For example, the antibodies of the present invention can also be generated using various phage display methods known in the art. In phage display methods, functional antibody domains are displayed on the surface of phage particles which carry the polynucleotide sequences encoding them. In a particular, such phage can be utilized to display antigen-binding domains expressed from a repertoire or combinatorial antibody library (e,g., human or murine). Phage expressing an antigen binding domain that binds the antigen of interest can be selected or identified with antigen, e.g., using labeled antigen or antigen bound or captured to a solid surface or bead. Phage used in these methods are typically filamentous phage including fd and M13 binding domains expressed from phage with Fab, Fv or disulfide stabilized Fv antibody domains recombinantly fused to either the phage gene III or gene VIII protein. Examples of phage display methods include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18 (1997); Burton et al., Advances in Immunology 57:191-280 (1994): PCT application No. PCT/GB91/01134; PCT publications WO 90/02809; WO 91/10737; WO 92/01047; WO 92/18619; WO 93/11236; WO 95/15982; WO 95/20401; and U.S. Pat.Nos. 5,698,426; 5,223,409; 5,403,434; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired BMP-2 binding fragment, and expressed in any desired host, including mammalian cells, insect cells, plant cells, yeast, and bacteria, e.g., as described in detail below. For example, techniques to recombinantly produce Fab, Fa and F2 fragments can also be employed using methods known in the art such as those disclosed in PCT publication WO 92/22324; Mullinax et al., BioTechniques 12(6): 864-869 (1992); and Sawai et al., AJR1 34:26-34 (1995); and Better et al., Science 240:1041-1043 (1988).

Examples of techniques which can be used to produce single-chain Fvs and antibodies include those described in U.S. Pat. Nos. 4,946,778 and 5,258,498; Huston et al., Methods in Enzymology 203:46-88 (1991); Shu et al, PNAS 90:7995-7999 (1993); and Skerra et al., Science 240:1038-1040 (1988). For some uses, including in vivo use of antibodies in humans and in vitro detection assays, it may be preferable to use chimeric, humanized, or human antibodies. A chimeric antibody is a molecule in which different portions of the antibody are derived from different animal species, such as antibodies having a variable region derived from a murine monoclonal antibody and a human immunoglobulin constant region. Methods for producing chimeric antibodies are known in the art. See e.g., Morrison, Science 229:1202 (1985); Oi et al., BioTechniques 4:214 (1986); Gillies et al., (1989) J. Immunol. Methods 125:191-202; U.S. Pat. Nos, 5,807,715; 4,816,567; and 4,816397. Humanized antibodies are antibody molecules from non-human species antibody that binds the desired antigen having one or more complementarity determining regions (CDRs) from the non-human species and framework regions from a human immunoglobulin molecule. Often, framework residues in the human framework regions will be substituted with the corresponding residue from the CDR donor antibody to alter, preferably improve, antigen binding. These framework substitutions are identified by methods well known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; Riechmann et al., Nature 332:323 (1988). Antibodies can be humanized using a variety of techniques known in the art including, for example, CDR-grafting (EP 239,400; PCT publication WO 91/09967; U.S. Pat. Nos. 5,225,539; 5,530,101; and 5,585,089), veneering or resurfacing (EP 592,106; EP 519,596; Padlan, Molecular Immunology 28(4/5): 489-498 (1991); Studnicka et al., Protein Engineering 7(6): 805-814 (1994); Roguska, et al., PNAS 91:969-973 (1994)), and chain shuffling (U.S. Pat. No. 5,565,332).

Completely human antibodies are particularly desirable for therapeutic treatment of human patients. Human antibodies can be made by a variety of methods known in the art including phage display methods described above using antibody libraries derived from human immunoglobulin sequences. See also, U.S. Pat. Nos. 4,444,887 and 4,716,111; and PCT publications WO 98/46645, WO 98/50433, WO 98/24893, WO 98/16654, WO 96/34096, WO 96/33735, and WO 91/10741.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring that express human antibodies. The transgenic mice are immunized in the normal fashion with a selected antigen, e.g., all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies, For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, see, e.g., PCT publications WO 98/24893; WO 96/34096; WO 96/33735; U.S. Pat. Nos. 5,413,923; 5,625,126; 5,633,425; 5,569,825; 5,661,016; 5,545,806; 5,814,318; 5,939,598; 6,075,181; and 6,114,598.

Completely human antibodies which recognize a selected epitope can be generated using a technique referred to as "guided selection." In this approach a selected non-human monoclonal antibody, e.g., a mouse antibody, is used to guide the selection of a completely human antibody recognizing the same epitope. (Jespers et al., Biotechnology 12:899-903 (1988)).

Regardless of the form of the binding antibody molecule chosen, the in vivo binding of the antibody to the target preferably occurs at a site that does not interfere with the biologic activity of the target, including receptor binding or other activity effectors. The molecular presentation of the antibody-antigen complex obtained on the collagen membrane carrier or the medical implant thus not only increased the localized concentration of the biomolecule, but also allowed desired normal and natural biological activity of the biomolecule in vivo, which enhanced the healing process for the implant procedure.

The antibody clones generated were tested in vivo for reactivity with the targeted biomolecule. Initial tests included binding with the modified immunized biomolecule, to test that the biologic activities of the captured molecule were accessible and were not neutralized.

Initial tests included reaction of biomolecules with specific antibodies at varying concentrations in a checkerboard format. Each of the antibodies which were found in the initial testing to react positively to the immunizing molecule were selected and reacted with that antigen to form an immune complex. The immune complexes generated were tested in an in vitro assay followed by an in vivo assay for biologic activity.

To obtain fragments of antibodies, the monoclonal antibodies were enzymatically digested to generate antigen-binding fragments, including Fab and F(ab)'2. The whole immunoglobulin molecules or their fragments may be used in in vitro and in vivo assays to select the most efficacious molecule with which to capture the native biomolecules. Alternatively, a method for generation of these fragments was to produce these fragments using recombinant technology. In this manner, smaller fragments such as ScFc or CDR were generated.

Various methods were used to attach the biomolecules to the surface of the medical, implant device, including adsorption, use of spacer molecules, silanization, glycosylation and covalent bonding, as discussed in more detail below. Adsorption of antigen-binding molecules to implantable devices was accomplished by incubation of implantable devices with antigen-binding molecules. For each procedure, the incubation period and conditions, such as pH, temperature and ionic concentrations were optimized for each antigen-binding molecule and the implantable device.

We tested a number of scaffolds for immobilization of antibodies for influence of scaffold on bone regeneration. Scaffolds included: absorbable collagen sponge derived from bovine Achilles tendon (ACS; Collacote, Integra Life Sciences, Plainsboro, NJ), bilayer non-crosslinked porcine collagen membrane (Bio-Gide®, Geistlich Biomaterials, Wolhusen, Switzerland), Bovine deproteinated cancellous bone (Bio-Gide®, Geistlich Biomaterials, Wolhusen, Switzerland), ß-Tricalcium Phosphate (Cerasorb®, RIEMSER Inc, Germany) and titanium implant (Osseospeed®, Astratech, Mölndal, Sweden). Comparison of the histologic
bone regeneration with each of the scaffold material demonstrated that ACS was associated with highest degree of bone regeneration (data not shown). This was perhaps due to the fact that ACS has the most rapid resorption rate of all scaffolds tested.

Currently, rhBMP-2 has been approved by the FDA to be used with ACS carrier. Therefore, we used collagen membranes (ACS) as the scaffold in the present study.

### Example 1: BMP-2 specific antibodies

Approximately two dozen commercially available anti-BMP-2 antibodies were tested, including monoclonal antibody clone 3G7S (IgG2a; Abnova, Taipei, Taiwan), monoclonal antibody clone 4B12 (IgG2a; Abnova), and polyclonal Ab (pAb) (Rabbit, rhBMP-2, Biovision, Mountain View, CA). In preliminary studies, three monoclonal antibody clones were tested, including mAb1 (clone 100221), mAb2 (clone 100230) and mAb3 (clone 65529). A polyclonal antibody specific for BMP-2 was also tested, pAb (affinity purified goat anti-BMP-2 antibody).

Additionally, a murine monoclonal antibody (mAb) library was created. Monoclonal antibodies specific for the biomolecule bone morphogenetic protein (BMP-2) were generated according to standard procedures (Galfre G and Milstein C, 1981, Methods Enzmol., 73 (Pt B): 3-46; Milstein C, 2003, Immunol. Today, Aug 21(8): 359-64). Mice were inoculated with an immunogenic dose of BMP-2 (RNV System, Medtronic) with an appropriate adjuvant. The hybridomas generated from the splenocytes of immunized animals were screened for those which bound BMP-2. From several thousand generated colonies, 480 picked colonies were screened by ELISA for BMP-2 binding; 37 clones were selected. Some clones lost expression, but 13 clones with high expression of BMP-2 were identified. Clones were generated using ClonaCell-HY hybridoma cloning kit (StemCell Technologies, Vancouver, BC, Canada) according to the manufacture's protocol. The isotyping of the screened clones were performed using Mouse MonoAB ID KIT-HRP (Invitrogen, Carlsbad, CA) as described in the manufacture's recommendation. BMP-2 specific antibody clones from this hybridoma library were labeled with a C prefix, such as C3 -C29, and tested for effect on bone regeneration.

The monoclonal hybridoma clones C22 and C13 were deposited in a tissue depository.

Example 2: In vitro capturing of BMP-2 using monoclonal antibodies immobilized on a culture dish.

An in vitro culture system was developed to determine the ability of anti-BMP-2 Ab's to capture BMP-2 from solution. Antibodies (25 µg/ml) diluted in carbonate/biocarbonate solution (0.5 mM, pH 9.5) were immobilized on 24-well culture dishes by overnight incubation at room temperature, followed by 6 washes with PBS. Recombinant human BMP-2 (rhBMP-2, Medtronic, Minneapolis, MN, 100 ng/ml) was then incubated at 4°C for 1 hour. Free rhBMP-2 was removed by 6 washes with PBS.

Preliminary tests included reaction of BMP-2 with specific antibodies at varying concentrations in a checkerboard format. Each of the antibodies that were found in the initial testing to react positively to the immunizing molecule were selected and reacted with that antigen to form an immune complex. The immune complexes generated were tested in an in vitro assay for osteogenic response.

### Example 3: in vitro assay of biologic activity of antibody-BMP immune complex by flow cytometric analysis

An in vitro flow cytometric assay was developed to determine if an immune complex between BMP-2 and an anti-BMP-2 antibody retained its ability to bind to the BMP-2 receptor on the cell surface of osteogenic cells. Generally, rhBMP-2 was incubated with various anti-BMP-2 antibodies and the immune complexes were incubated with C2Cl2 cells, an osteogenic cell line that expresses BMP receptors. This was followed by immunofluorescent labeling with phycoerythrin (PE)-conjugated goat anti-mouse Ab (Becton Dickinson, San Jose, CA). The intensity of fluorescent labeling was determined by measuring mean fluorescent intensity (MFI) by a flow cytometer (FACSCalibur, Becton Dickinson).

Osteogenic cell lines: Lines of cells with the potential to develop into osteoblast cells were used. Cells of the mouse myoblast cell line C2C12, mouse ostoblast cell line MC3TC-E1, and human osteoblast cell line hFOB 1.19 were obtained from American Type Culture Collection (ATCC, Manassas, VA). C2Cl2 cells cultured in Dulbecco's Modified Eagle's Medium (DMEM, Sigma-Aldrich, St. Louis, MO) supplemented with 100 units/ml penicillin, 100 µg/ml streptomycin (Sigma-Aldrich), and 10% fetal bovine serum (FBS, Biocell Laboratories, Rancho Dominguez, CA). MC3T3-E1 cells were grown in alpha-MEM containing L-glutamine, ribonucleosides, and deoxyribonucleosides (Cat. No. 12571, Invtrogen, Grand Island, NY) supplemented with 100 units/ml penicillin, 100 µg/ml streptomycin (Sigma-Aldrich), and 10% FIBS (Biocell Laboratories). hFOB 1.19 cells were cultured in 1:1 mixture of Ham's F12 Medium and DMEM (Cat. No. D2906, Sigma) supplemented with 2.5 mM L-glutamine 100 units/ml penicillin, 100 µg/ml streptomycin (Sigma-Aldrich), and 10% FBS (Biocell Laboratories). The cells were cultured in each media at 37°C (C2C12 and MC3T3-E1) or 34°C (hFOB 1.19) in a humidified atmosphere of 5% CO₂ in air.

In the flow cytometric analysis, a BMP-2 specific antibody (see Example 1) was incubated with rhBMP-2 at 4°C for 30 min at saturating antibody concentrations. Then, the immune complexes were incubated with C2Cl2 cells for 20 min at 4°C. Unbound antibody-BMP was removed by washing with PBS. Cells were labeled by immunofluorescent labeling with immunofluorescent labeling with phycoerythrin (PE)-conjugated goat anti-mouse Ab (Becton Dickinson, San Jose, CA). The intensity of fluorescent labeling was determined by measuring mean fluorescent intensity (MFI) by a flow cytometer (FACSCalibur, Becton Dickinson).

Results of flow cytometric analyses of various BMP-2 specific antibodies are shown in Figure 6. The antibodies tested included a panel of monoclonal antibodies (see Example 1), as well as several polyclonal antibodies. After saturation binding with rhBMP-2, the immune complexes were incubated with C2C12 cells, followed by immunofluorescent labeling with fluorochrome-conjugated goat anti-mouse Ab to detect cells that bound a BMP-2: antibody immune complex. Controls included membrane carrier only (-), lack of second anti-mouse antibody, and isotype matched IgG antibody not specific for BMP-2 (Ablso). Results were representative of three independent experiments. Two-tailed T-test was used to determine significance at p < 0.05 (*), or p<.0.01.(#).

Results in Figure 6 demonstrated binding of the immune complexes to the C2C12 cells of only a few anti-BMP-2 antibody clones. Compared with controls, there was significant binding of immune complexes of only some anti-BMP-2 antibodies (pAb, C15, C18, C21, C22, C24 and 3G7) at level of significance (*) p < 0.05. Other clones as C3, C6, C9, C13, C20, C26, C29 showed little binding. The only two clones with significant ability to bind BMP-2 while not interfering with its binding to the BMP receptor-positive cells were the clones 3G7 (IgG2a) and C22 (IgM) at (#) p< 0.01. Interestingly, an affinity purified polyclonal rabbit anti-human BMP-2 also was able to bind BMP-2 and allowed BMP binding to target osteogenic cells.

These results (Figure 6) demonstrated that compared with controls, most of the antibody clones did not have significant ability to bind BMP-receptor positive cells. Only a few monoclonal antibody clones had significant binding capability with C2Cl2 cells when complexed with rhBMP-2. These results suggested that some monoclonal antibodies can bind BMP-2 and allow BMP-2 binding to the BMP-receptor of cells, while other monoclonal antibodies may prevent binding of BMP-2 to its receptor. This suggested the need to test the model of favorable and unfavorable antibody presentation (see Figure 2), as previously discussed in experimental strategy. We have devised an in vitro assay to assess the ability of various antibodies to mediating osteogenic differentiation (Figures 7 and 8). However, the most significant testing performed has been the in vitro bone regeneration of critical size calvarial defects (Figures 9 to 13).

### EXAMPLE 4: Alkaline phosphatase assay, in vitro assay for osteogenic differentiation by BMP-2 immune complexes

An in vitro cell culture system was developed to determine the ability of anti-BMP-2 Ab's to capture BMP-2 from solution and to mediate osteogenic differentiation of undifferentiated osteogenic cells. Because the BMP-2 signaling cascade is known to increase phosphorylation of proteins, the quantitation of phosphatase activity correlates with BMP-2 osteogenic activity. In this way, the osteoblastic differentiation of cells incubated with BMP-2 specific antibody immune complexes under in vitro culture conditions was determined by an alkaline phosphatase activity assay.

Antibodies (25 µg/ml) diluted in carbonate/biocarbonate solution (0.5 mM, pH 9.5) were immobilized on 24-well culture dishes by overnight incubation at room temperature, followed by 6 washes with PBS. Recombinant human BMP-2 (rhBMP-2, Medtronic, Minneapolis, MN, 100 ng/ml) was then incubated at 4°C for 1 hour. Free rhBMP-2 was removed by 6 washes with PBS. Cell lines with potential to differentiate into osteoblasts (C2C12, MC3T3 E1 or hFOB cell lines) were then added to the plates containing the immobilized Ab-BMP-2 immune complexes. The cells were cultured for two days. The osteoblastic differentiation of cells in the cultures was determined by measurement of alkaline phosphatase activity. Controls included omission of antibody or rhBMP-2 or replacement of specific Ab with isotype-matched antibodies. Positive control included rhBMP-2 at 200 ng/ml in solution. Initial dose-response studies demonstrated that 10 ng/ml of rhBMP-2 in the absence of anti-BMP-2 antibody when incubated with culture plates did not induce osteoblastic differentiation of culture cells (data not shown). Therefore, 10 ng/ml of rhBMP-2 was selected as the sub-osteogenic concentration in these studies.

C2C12 cells were maintained at 37 °C in a 5% CO₂ humidified incubator in DMEM, supplemented with penicillin, streptomycin, and 10% FBS. Cells were subjected to differentiation under low serum conditions. C2C12 cells were plated at 3 × 10⁴ cells/well in 94-well plates in 50 µl of differentiation media (DMEM with penicillin, streptomycin, and 5% FBS). Four days later, alkaline phosphatase activity from each groups were investigated. Briefly, for quantitative alkaline phosphatase assays, triplicate wells were washed twice in 1X PBS and lysed for 20 min at RT by shaking in 150 µl of cell lysis buffer (0.2% Triton X-100 in normal saline). The adherent cells were scrapped off using yellow tip and transferred into an eppendorf tube. The cell suspension was centrifuged at 2,500 X g (3,726 rpm, S4180 rotor in CS-15R centrifuge, BECKMAN) for 10 min at 4°C. The supernatants were transferred into new eppendorf tubes. The alkaline phosphatase activity was measured using a pNPP-based method (Sigma Fast™ p-nitrophenyl phosphate tablets, Sigma-Adrich). Protein concentration was measured by Bradford Method using Protein Assay Kit (Cat. No. 500-0006, Bio-Rad Laboratories, Hercules, CA) as described as the manufacture's protocol. The enzyme activity was normalized to total cellular protein.

Figure 7 shows results of testing for alkaline phosphatase activity of immobilized immune complexes for three BMP-2 specific monoclonal antibodies and one polyclonal antibody clone. Antibodies alone or 10 ng/ml of rhBMP-2 alone did not induce significant osteogenic differention of hFOB cells. When immobilized on plates and bound with BMP-2, three monoclonal antibody clones specific for BMP-2 (mAb1, clone 100221; mAb2, clone 100230; mAb3, clone 65529) and one polyclonal clone (pAb, affinity purified goat anti-BMP-2 polyclonal antibody) induced significant osteogenic differentiation in subsequently attached hFOB cells, as indicated by an increase in alkaline phosphatase activity.

Figure 8 shows a study similar to that shown in Figure 7 using the C2C12 cell line and other BMP-2 specific monoclonal antibodies generated from a hybridoma library (see Example 1). Culture plates were coated with saturation concentration of anti-BMP-2 polyclonal (pAb) or monoclonal antibodies, and then blocked with BSA at 5 mg/ml. rhBMP-2 at 100 ng/ml was then added followed by extensive washes to remove free BMP-2. C2C12
cells were added and cultured for 2 days. The alkaline phosphase activity (ALP) was determined as described above. Results confirmed that monoclonal antibodies 3G7 and C22 were able to mediate osteogenic differentiation in the presence of 10 ng/ml of rhBMP-2. These data demonstrated that the in vitro osteogenic differentiation assay may be used as an assay to screen the suitability of antibodies which are capable of mediating AMOR.

### EXAMPLE 5: in vivo bone regeneration: calvarial defect model

The calvarial defect model (Cowan et al., 2004) was employed in both rats (Figures 9 and 11) or rabbits (Figure 18, 19) to determine the effects, of various BMP-2 antibodies on bone heating and regeneration. Generally, BMP-2 specific antibodies were immobilized on absorbably collagen sponge (ACS),implanted into calvarial defects and assayed for bone growth by micro-computed tomography, histology and histomorphmetric analyses. Controls included no fill (-), isotype-match antibody absorbed on membrane, or membrane only (collagen).

Calvarial defects were created by removal of portions of bone from the calvaria, the dome-like portion of the skull, of 8-weeks old rats in sterile conditions and under general anesthesia using xylazine and ketamine. Full thickness skin flaps were raised and the left and right parietal bones were exposed. Four, five or eight mm diameter defects in parietal bones were generated using a hand drill trephine burr for consistency. Constant saline irrigation was conducted during the procedure. Following creation of the calvarial defect, the skin was resealed. Following surgery at designated time intervals, live animals were either scanned with micro-computed tomography (µ-CT), or were sacrificed for subsequent histological and histomorphometry analyses to determine tissue appearance and quantitation of bone fill in the calvarial defect regions.

Micro-computed tomography, Live animals were scanned with micro-computed tomography (µ-CT) at 2, 4 or 6 weeks after surgery. Each rat was placed in a sample holder in the cranial-caudal direction and scanned using a high-resolution micro-CT system (MicroCAT II, Siemens Medical Solutions Molecular Imaging, Knoxville, TN) at a spatial resolution of 18.676 µm (Voxel dimension) 1,536 × 1,536 pixel matrices. Rats were maintained under general anesthesia with isoflurane during the scanning procedure. After scanning, the 2D image data was stored in the Digital Imaging and Communications in Medicine (DICOM) format, transferred to a computer, and a 3D reconstruction and analysis were performed. In order to reduce the size of data for computation, the calvarial region was cropped and saved from the obtained consecutive microtomographic slice images as a volume of interest (VOI) using Amira software (Visage Imaging, San Diego, CA). In this step, the original spatial resolution was maintained, because data were not resampled. The volume of new bone in calvarial defect was measured using V-Works 4.0 software (Cybermed Inc., Seoul, Korea). The bone tissue was segmented using a global thresholding procedure. New bone was separated from pre-existing bone by applying a cylindrical divider whose base is same as the defect, and the volume was calculated.

Histological analyses: Calvarial specimens were fixed with 10% neutral buffered formalin (Richard-Allan Scientific, Kalamazoo, MI) for 24 h at 4°C. Tissues were then decalcified in Decalcifying solution (Richard-Allan Scientific) for 2 days at 4°C. The samples were dehydrated in graded alcohol and embedded in paraffin. 5 µm sections were stained with hematoxylin and eosin (H&E) and Masson Trichrome (tri) (Sigma) for morphology evaluation.

Histomorphometry : New bone of calvaria defects of animals was dehydrated in graded ethanol (70%, 95%, 100%) at 4°C, defatted in acetone and infiltrated in a liquid methyl methacrylate monomer (Koldmount™ Cold Mounting Liquid, Mager Scientific). The bone samples were then embedded in methyl methacrylate (Koldmount™ Cold Mounting Kit, Mager Scientific) and sectioned using a low-speed sectioning saw (South Bay Technology, Model 650, San Clemente, CA) with a diamond wafering blade (Mager Scientific, Dexter, MI). Sections 200 µm thick were made at the mid-diaphysis, 1 to 2 mm proximal to the TFJ, and were hand ground and polished to a final thickness of between 50 and 75 µm using wet silicon carbide abrasive discs. Sections were imaged using the Nikon DAPI-FITC- All histomorphometric analyses were performed using standard ASBMR methods and nomenclature.

### Example 6: in vivo bone regeneration in rat calvarial defects filled with immobilized BMP-2 specific antibodies: two weeks post-surgery

To investigate the ability of specific anti-BMP-2 antibodies to mediate AMOR in vivo, the calvarial defect model was utilized. The osteogenic responses of the BMP-2 monoclonal antibodies (tested in Example 1) were tested in vivo in 4-6 week old rats at two weeks after surgery. BMP-2-specific monoclonal antibodies (described in Example 1) or isotype control antibodies (negative control) were immobilized onto absorbable collagen sponge (ACS) by incubation overnight at 4°C. A variety of other carriers were tested, including collagen membranes (Biogide or Collacote), bovine Inorganic bone (BioOss) and microbeads (Dynabeads). Four mm defects in size were created surgically in the calvaria of 4-6 week-old rats and then treated as controls by leaving the defect unfilled, or by filling with membrane carrier alone. Alternatively, the defect was filled with each of four experimental BMP-2 monoclonal antibodies immobilized on collagen membranes. A total of 3 rats were used. Various BMP-2 specific antibodies (Example 1) were immobilized by absorption on absorbable collagen sponge (ACS) by overnight incubation at 4°C in carbonate buffer. The antibodies used included anti-BMP-2 polyclonal Ab (pAb), several mAbs, as well as isotype-matched Ab. The rats were allowed to heal for a period of 2 weeks. The calvarial defect regions were scanned by µ-CT, and then the rats were sacrificed by intraperitoneal injection of nembutal. Histological examination and histomorphometry was then performed to evaluate percentage bone fill.

Results in Figure 9 (panels A-F)show the µ-CT and histological data obtained in rats two weeks after surgery testing the potential of BMP-2-specific antibodies using the calvarial defect model for in vivo testing of osteogenesis. Controls: calvarial defects were left unfilled (A) or filled with membrane carrier only (B). Experimentals: calvarial defects were filled with various BMP-2 specific antibodies absorbed to collagen membranes (C -F). The following antibodies described in Example 1 were absorbed to collagen membranes: mAb1 (C); pAb (polyconal antibody) (D); mAb2 (E) and mAb3 (F). Two weeks after surgery, rats were scanned by µ-CT, and then sacrificed for histological and histomorphometry analyses.

FIGURE 9, row (i) shows cross-section µ-CT scans, row (ii) shows coronal sections of µ-CT scans and row (iii) showing detail of the coronal scan. In the µ-CT scans, radiolucency (dark area) indicates a vacancy, while radio-opacities are lighter because of presence of dense tissue.

For controls, when the calvarial defects were left un-filled (panel A), the defect persisted as shown by dark areas, illustrating very low spontaneous healing within these critical size defects. When the defects were filled with collagen with isotype control antibodies (B), a thin layer of material (residual collagen membrane) persisted in cross-section and exhibited low density. The coronal µ-CT scan shows the defect area to be lightly filled.

Examination of panels C - F in Figure 9 shows that when calvarial defects were filled with BMP-2-antibodies immobilized on collagen membranes, all showed increased tissue mass, greater than that obtained in the controls (panel A-B). Increased bone density was observed as evident by the dense, filled in regions in µ-CT scans in both cross-sectional (row i) and longtitudinal scans (rows ii and iii). The fill obtained was evident in the corresponding cross-sectional views. Comparison of the coronal µ-CT scans for panels C - F confirmed the results from cross-sectional data. The most fill was obtained for BMP-2 hybridoma mAb1 and mAb3.These µ-CT data demonstrated that when the defects were filled with BMP-2-immobillized collagen membranes, enhanced results for bone density were found after only two weeks of healing time. (mAb1, clone 100221) and one polyclonal AB (pAb, affinity purified goat anti-BMP-2 polyclonal antibody).

Histologic examination of the calvaria defects provided additional information on formation of new bone (Figure 6, row iv). For the controls, these data demonstrated only limited osteogenesis potential, i.e., defects left unfilled, or those filled with isotyope control antibody-treated collagen membrane, these data demonstrated only limited osteogenesis otential since most of the cavarial defect was filled with connective tissue. On the other hand, defects filled with BMP2-treated collagen membranes exhibited increased osteogenesis as indicated by higher percentage of the defect being filled with vital bone. In all four of the BMP-2 specific antibody preparations tested (mAb1, pAb, mAb2 and mAb3), a continuous layer of new bone was found on the dural side of the defects. The collagen membrane was a dense porcine collagen (Bio-Gide; Osteohealth, Shirley, New York). (mAb1, clone 100221; mAb2, clone 100230; mAab3, clone 65529).

Corresponding results of histomorphometry analyses for this experiment are shown in FIGURE 10. The results of measurement of percentage bone fill confirmed the µ-CT scan data observed in Figure 9. All three of the BMP-2 monoclonal antibodies (mAb1, mAb2 and mAb3) had greater percentage bone fill than controls. The percent bone fill for pAb was slightly less than control values. Of the three monoclonal antibodies tested in this experiment, BMP-2 specific monoclonal antibody mAb1 showed the greatest percentage bone fill of the four experimentals tested.

### Example 7: in vivo bone regeneration in rat calvarial defects filled with immobilized BMP-2 specific antibodies: two, four and six weeks post-surgery

Using techniques as described in Example 5 -6, other BMP-2 specific antibodies immobilized on absorabable collagen sponge (ACS; Collacote, Tutogen Medical distributed by Zimmer Dental, Carlsbad, California)were implanted in rat calvarial defects. BMP-2specific antibody clones included pAb, C3, C7, C9, C13, C21, C22, C24, 4B17 and 3G7S (Example 1). Live animals were scanned with micro-CT at two, four and six weeks after surgery, and then were euthanized at six weeks. As described in Example 5 -6, calvarial specimens were then harvested, paraffin-embedded and sectioned:-Histological staining with hemotoxylin and eosin (H & E) and trichrome (tri) was performed, as well as histomorphometry analysis to evaluate bone regeneration by the percentage of bone fill.

Figure 11 shows the µ-CT data obtained for three significantly responding monoclonal clones (3G7S, 4B12 and C22) that mediated a favorable bone regeneration, a monoclonal clone that failed to mediate bone regeneration(C13), and controls of unfilled defects (-) or isotype matched antibodies. Results for in vivo measurement of osteogenic differenation of clones 3G7S, 4B12, C22 and C13 in rats at two, four and six week points are shown with controls in Figures 11A - 11N. Representative calvarial specimens are shown. Figure 11M shows data aligned on a single page for better comparison.

When other BMP-2 specific antibodies were tested (Example 1), marked differences in osteogenic reactions of the BMP-2 antibody clones again were observed. Results demonstrated significantly more bone fill when defects were filled with specific clones of anti-BMP-2 antibody (3G7S, 4B12 and C22) immobilized on collagen membranes, than with other antibody clones. Each of these clones generated significantly more new bone tissue when compared with controls. On the other hand, many monoclonal antibody clones demonstrated little osseous activity. For example, the C13 clone showed little osteogenic differentiation, similar to that observed in controls.

Figure 12 shows the µ-CT analysis obtained at three time points of two weeks, four weeks and six weeks for the BMP-2 specific antibody clones tested. Figure 13 shows the histomorphometric quantification of percentage osteoid bone fill. Antibody clones 4B12, 3G7S, C21 and C22 demonstrated greater amount of bone density and percentage of bone fill than other BMP-2 specific antibody clones. Clones C3 and Clone13 had low levels of bone fill, similar to that of controls.

To summarize, the antibody clones with the highest ability to mediate osseous regeneration included C3, C7, C21, C22, 3G7 and 4B12. In particular, the degree of bone fill with clones C22 (IgM), 3G7 (IgG2a) and 4B12 (IgG2a) has consistently surpassed other BMP-2 specific clones in our experiments. Conversely, clones C9, C 13, C15, C18, C19, C20, C24 and C29 consistently failed to promote bone regeneration in vivo.

### Example 8: In vitro capture of BMP-2 from bone homogenate by BMP-2 specific antibodies

The extent of BMP-2 capture from bone by specific antibodies in vitro was determined. Isotypic antibody or BMP-2 antibody clones (4B12, 3G7S or pAb) were coupled to tosyl-activated beads. Functionalized beads were incubated with human bone homogenates. Proteins were then eluted from the beads, resolved on SDS-polyacrylamide gel electrophoresis and probed with labeled anti-BMP-2 antibody. Results are shown in Figure 14. Significant amounts of BMP-2 were captured by BMP-2 specific antibodies attached to beads. Quantitative measurement of the density in lane 3 (3G7S clone) (214.6) versus that for the 2 ng of rhBMP-2 loaded directly on the gel (22.5), allowed estimation that approximately 19ng of BMP-2 was captured from bone by the 3G7S antibody clone.

### Example 9: In situ distribution of BMP-2 and osteocalcin expression by immunohistochemisty

Immunohistochemistry (IHC) was used to examine the in situ distribution of expression of BMP-2 (Figure 15) and osteocalcin (Figure 16). Because osteocalcin is a hormone produced by osteoblastic cells, it would be expected that active osteoblastic cells would express osteocalcin. Specimens procured from rat calvarial defects implanted with antibodies immobilized on collagen membranes that were described in Example 6 were labeled with anti-BMP-2 polyclonal antibody as the primary Ab to detect the expression of BMP-2 antigen in situ. The antibodies used include BMP-2 specific monoclonal clones C13, 3G7S, 4B12, C21 and C22. Sections were labeled with anti-BMP-2 polyclonal antibody followed by HRP-conjugated secondary antibody. Similarly, sections were labeled to detect osteocalcin. A enlarged detail for each histological section is indicated by an arrow in Figures 15 - 16. Figure 17 shows the sections aligned on a single page for comparison.

Results shown in 15A -15B revealed intense staining for expression of BMP-2 in sites implanted with the antibody clones that were previously shown to mediate bone regeneration (3G7S, 4B12, C21 and C22). In contrast, the BMP-2 specific antibody clone that consistently did not mediate bone regeneration (C13) also showed significantly less BMP-2 labeling. Similar results of a lower amount of BMP-2 labeling were also found for other antibody clones that were consistently not associated with significant bone regeneration (C9, C13, C15, C18, C19, C20, C24 and C29) (data not shown).

To determine the extent of osteoblastic activity, the expression of osteocalcin was also measured in simlar samples by immunohistochemistry. Results are shown in Figure 16A-16B. As with BMP-2 expression, the amount of labeling was greatly increased for the four BMP-2 specific antibody clones (C21, C22, 3G7S and 4B12) as compared with controls and with the C13 hybridoma clone.

It is important to note that all anti-BMP-2 Ab clones generated in the murine monoclonal library (see Example 1) have been selected because of their high affinity for rhBMP-2. In theory, all monoclonal antibody clones used should equally capture BMP-2 in vivo. Therefore, the fact that more BMP-2 labeling was noted in conjunction with sites that have favorable bone regeneration and greater expression of osteocalcin suggests that the BMP-2 captured by some Ab clones may lead to bone regeneration, while other monoclonal antibody clones do not favor bone regeneration. In those sites where significant bone regeneration occurred, there is likely more BMP-2 detection as a result of more active osteoblastic activity.

### Example 10: in vivo bone regeneration following implantation of immobilized anti-BMP-2 antibodies in rabbit calvaria: two, four and six weeks post-surgery

To investigate the ability immobilized anti-BMP-2 Ab's to mediate bone regeneration in other species, the calvarial defect model utilized for rats (Examples 5 - 7) was repeated in 6 month old rabbits. Eight mm surgical defects were created and implanted with absorbable collagen sponge (ACS) alone or with immobilized antibodies. The antibodies used included isotype match antibody (iso), anti-BMP-2 polyclonal Ab (pAb8) or various anti-BMP-2 monoclonal antibody clones. Rabbits were euthanized at 4 weeks and calvaria were harvested. Specimens were scanned with µ-CT, followed by histological staining with H&E and trichrome. Histomorphometry was performed. T-test relative to isotype-matched antibody indicated significance p< 0.005 (*).

The results for bone regeneration in rabbit calvarial filled with immobilized BMP-2 specific antibodies are shown in Figure 18 - 19. Figure 18 shows µ-CT scans of cross-sections (Figure 18A) and coronal sections (Figure 18B) of calvarial defects filled with collagen membrane immoblilized with either of two BMP-2 specific antibody clones (C20 and C22), as compared with istotypic matched antibody controls. Limited bone fill iwas observed in rabbit calvarial defects for clone C20 and for the isotype control. Alternatively, the clone C22 showed significant increase in bone fill on both cross-sectional and coronal scans.

Figure 19 shows the corresponding histological examination. The increase in bone tissue mass is clearly evident for the C22 clone, but not for the conrol or the C21 monoclonal antibody clone.

Figure 20 shows the histomorphometric analysis of the percentage bone fill obtained by various antibody clones and controls in rabbit calvarial defects. Of the monoclonal antibody clones tested in rabbits, clones C22 and 3G7S exhibited significant ability to yield successful bone regeneration in calvarial defects (p< 0.005). Conversely, clones C9, C13, C15, C18, C19, C20, C24 and C29 again failed to promote bone regeneration in vivo.

### Example 11: Enrichment of BMP-2 specific antibodies having heparin epitope

The BMP-2 molecule has a heparin-binding domain (HBD), as well as a wrist and knuckle domains (Figure 4) (S. Daopin, K. A. Piez, Y. Ogawa, and D. R. Davies, Crystal structure of transforming growth factor-beta 2; an unusual fold for the superfamly. Science, Vol. 257, Issue 5068, 369-373).The wrist and knuckle domains are thought to be responsible for engagement of the receptors BMP-R1 and BMP-R2, respectively. The HBD does not appear to be involved in binding to the BMP-2 receptors, and BMP-2 bound to heparin appears to have higher biologic activity (Zhao et al., J. Biol. Chem. 281: 23246-23253, 2006). Therefore, it was reasoned that if a polyclonal anti-BMP antibody, which includes immunoglobulins with specificity against various BMP epitopes, was passed through an affinity column that has BMP-2 bound to heparin sulfate, then those anti-BMP-2 antibodies with specificities against all epitopes other than the HBD could be removed. By isolating the BMP-2 antibody from the BMP-2: heparin column, there should be an enrichment for antibodies specific for HBD or epitopes which are sterically hindered while BMP is bound to heparin.

BMP-2 was first bound to a heparin sulfate column, and then a polyclonal antibody (clone pAb) (Example 1) was passed through the column. The resultant heparin-absorbed pAb was then removed by washing the column. The antibody fraction (AB-pAb) was tested in the rat calvarial model as described in Examples 5- 7. Samples included: controls of defects filled with collagen alone(collagen) or with isotype matched antibody immobilized on collagen membranes (collagen-isotype), collagen membranes with absorbed polyclonal antibody (pAb) that was not placed on the heparin-sulfate column, and the heparin-enriched antibody (AB-pAb) eluted from the BMP-2: heparin sulfate column. Micro-CT scans were taken at two and four weeks. At four weeks, animals were sacrificed and histological and histomorphometric analyses were made.

Results for heparin epitope enrichment of BMP-2 antibody binding are shown in Figure 21 and Figure 22. A comparison of µ-CT scans obtained from rat calvarial defects filled with pAb clone at two and four weeks (Figure 21A and C) were less than defects filled with polyclonal antibody with heparin binding enrichment (Figure 21B and 21D). At 4 weeks, the polyclonal anti-BMP2 antibody enriched for HBD epitope (Figure 21D) had a markedly increased bone regenerative biological activity as compared with pAb clone or controls. These results showed that the heparin-enriched polyclonal antibody when immobilized on collagen sponge, was able to lead to a nearly complete calvarial defect fill.

Figure 21E shows the histomorphometric quantitation of regenerated bone volume for this study. The heparin-enriched pAb antibody fraction had more bone fill than the pAb clone or for control (collagen). Figure 22 shows the histological staining obtained for controls and experimental samples. Although pAb has greater bone regeneration than either of the two controls (collagen) and (isotype controlled antibody), the heparin-absorbed pAb clone demonstrated a marked increase in bone regeneration as indicated by increased volume and density of tissue mass and cell number and new bone formation.

### EXAMPLE 12. Osteogenesis on and around dental implants in rabbit tibia.

BMP-2-specific monoclonal antibody or isotype control monoclonal antibody were adsorbed on dental implants (Astratech 3.5 x 8.0 mm dental implants with Fluoride-modified surface). Antibody-treated implants were surgically inserted in the tibia of rabbits and were allowed to heal for 2 to 6 weeks. Following the healing intervals, animals were sacrificed and the tibia were harvested. Some of the implants were removed for scanning electron microscopy (SEM) (Figure 23). The tibia containing implants were imaged with µ-CT. Blocks of tibia containing implants were also subjected to histologic examination.

Results in Figure 23 show SEM images of an implant coated with isotyope control monoclonal antibody (negative control, A,C,E) and one coated with BMP-2-specific monoclonal antibody (mAb1, clone 100221) (experimental, B,D,F) (clone 100221, R&D Systems; www.rndsystems.com). The experimental implant exhibited higher layers of adherent cells in the micro-thread, as well as macro-thread areas.

The µCT data in Figure 24 demonstrated higher density of bone around the experimental implant threads (B), compared with the control (A). As shown in the bar graph in panel C, the quantitation of the density around the threads in Houndsfield units showed a significantly higher bone density (p < 0.05) around the macro-threads of the implant coated with BMP-2 specific monoclonal antibody, as compared with the control.

As shown in Figure 25, the histological results of the rabbit tibia implants also demonstrated increased bone regeneration in between the threads of the implant coated with BMP-2 specific monoclonal antibody, as compared with the controls. The bone-to-implant contact area also appeared greater in the experimental implant than in control. These observations were made in both the micro- as well as macro-thread areas.

As noted above, a variety of medical implants are used when the process of osseointegration promotes healing. These implants include a variety of biocompatible structures designed to engage the skeletal structure of the body to replace or support a bone structure, including specifically dental implants, craniofacial structures and bone and joint replacement component parts. Medical implants are made of a wides selection of biocompatible materials including titanium, titanium alloys, stainless steel, cobalt chromium alloys and amorphous alloys and synthetics, including composites and polymers such as PEEK, UHMWPE, and can include materials such as endogenous bone, cortical, cancellous, allograft, autograft, xenografts or deminerralized or partially demineralized bone.

## Claims

1. A pharmaceutical composition for use as a medicament in mediation of bone regeneration within a host comprising:
an antibody or antigen-binding portion thereof, that specifically binds to bone morphogenetic protein 2 (BMP-2) antigen,
wherein the antibody binds an epitope separate from a BMP-2 receptor-binding domain, and,
wherein the antibody BMP-2 complex mediates osteogenic differentiation.

2. The composition for use according to claim 1 wherein the antibody BMP-2 complex mediates osteogenic differentiation by signal transduction.

3. The composition for use according to claim 1 wherein the antibody BMP-2 complex binds a BMP receptor on an osteoprogenitor cell.

4. The composition for use according to claim 1 wherein a complex of the antibody and endogenous BMP-2 binds a BMP receptor on an osteoprogenitor cell and enhances the bone formation.

5. The composition for use according to claim 1, wherein the antibody increases the availability of endogenous BMP-2 proteins surrounding the cell.

6. An antibody for use as a medicament in mediation of bone regeneration with properties wherein:
the antibody binds to an epitope of endogenous bone morphogenetic protein 2 (BMP-2),
wherein the antibody binds the BMP-2 epitope remote from BMP-2 receptor binding domains, and
wherein a complex of the antibody with endogenous BMP-2 promotes the formation of bone involved in osseointegration or osteogenesis; and optionally wherein at least a portion of a constant region is humanized, or at least a portion of the heavy chain is humanized.

7. The antibody for use according to claim 6 wherein binding of the antibody transduces intracellular signals.

8. The antibody for use according to claim 6 that is fully human.

9. A medical implant comprising
a biocompatible implant structure adapted to engage a skeletal structure of the body; an antibody coated on the implant structure, wherein the antibody specifically binds an epitope of bone morphogenetic protein, binds to the BMP-2 epitope remote from BMP-2 receptor binding domains and the antibody BMP-2 complex mediates osteogenic differentiations, and optionally wherein the antibody is coated on the implant with a synthetic linker, or the antibody is coated on the implant structure by adsorption.

10. The implant of claim 9 wherein an antibody BMP-2 complex mediates osteogenic differentiation by signal transduction.

11. The implant of claim 9 wherein the antibody BMP-2 complex binds a BMP-2 receptor on an osteoprogenitor cell.

12. The implant of claim 9 wherein the antibody BMP-2 complex enhances bone formation.

13. The implant of claim 9 wherein the antibody increases the availability of endogenous BMP-2 protein surrounding the cell.

## Patentansprüche

1. Pharmazeutische Zusammensetzung zum Gebrauch als Medikament bei der Vermittlung von Knochenregenerierung in einem Wirtskörper, Folgendes umfassend:
einen Antikörper oder Antigen-bindenden Abschnitt davon, der speziell an das knochenmorphogenetische Protein 2-Antigen (BMP-2) bindet,
wobei der Antikörper an ein Epitop bindet, das von einer BMP-2-bindenden Domäne getrennt ist, und,
wobei der Antikörper-BMP-2-Komplex osteogene Differenzierung vermittelt.

2. Zusammensetzung zum Gebrauch nach Anspruch 1, wobei der Antikörper- BMP-2-Komplex osteogene Differenzierung durch Signaltransduktion vermittelt.

3. Zusammensetzung nach Anspruch 1 wobei der Antikörper-BMP-2-Komplex einen BMP-Rezeptor an eine Osteoprogenitorzelle bindet.

4. Zusammensetzung nach Anspruch 1, wobei ein Komplex des Antikörpers und ein endogenes BMP-2 einen BMP-Rezeptor an eine Osteoprogenitorzelle binden und die Knochenbildung fördern.

5. Zusammensetzung nach Anspruch 1, wobei der Antikörper die Verfügbarkeit von endogenem BMP-2-Protein in der Umgebung der Zelle erhöht.

6. Antikörper zum Gebrauch als Medikament bei der Vermittlung von Knochenregenerierung mit Eigenschaften, wobei:
der Antikörper an ein Epitop des endogenen knochenmorphogenetischen Proteins 2 (BMP-2) bindet,
wobei der Antikörper das BMP-2-Epitop entfernt von der BMP-2-Rezeptor bindenden Domäne bindet, und
wobei ein Komplex des Antikörpers mit endogenem BMP-2 die mit der Osseointegration und der Osteogenese verbundene Knochenbildung fördert; und optional wobei wenigstens ein Abschnitt einer konstanten Region humanisiert ist oder wobei wenigstens ein Teil der schweren Kette humanisiert ist.

7. Antikörper zum Gebrauch nach Anspruch 6, wobei das Binden des Antikörpers intrazellulare Signale transduziert.

8. Antikörper zum Gebrauch nach Anspruch 6, der vollständig human ist.

9. Medizinisches Implantat, Folgendes umfassend
eine biokompatible Implantatanordnung, angepasst, um mit einer körperlichen Skelettstruktur in Eingriff zu treten;
einen auf der Implantatanordnung aufgetragenen Antikörper, wobei der Antikörper spezifisch an ein Epitop eines knochenmorphogenetischen Proteins bindet, entfernt von an BMP-2-Rezeptor bindenden Domänen an das BMP-2-Epitop bindet, und wobei der Antikörper-BMP-2-Komplex osteogene Differenzierungen vermittelt, und optional wobei der Antikörper mit einem synthetischen Linker auf dem Implantat aufgetragen wird, oder wobei der Antikörper durch Adsorption auf die Implantatanordnung aufgetragen wird.

10. Implantat nach Anspruch 9, wobei ein Antikörper-BMP-2-Komplex osteogene Differenzierung durch Signaltransduktion vermittelt.

11. Implantat nach Anspruch 9, wobei der Antikörper-BMP-2-Komplex einen BMP-2-Rezeptor an eine Osteoprogenitorzelle bindet.

12. Implantat nach Anspruch 9, wobei der Antikörper-BMP-2-Komplex die Knochenbildung fördert.

13. Implantat nach Anspruch 9, wobei der Antikörper die Verfügbarkeit von endogenem BMP-2-Protein in der Umgebung der Zelle erhöht.

## Revendications

1. Composition pharmaceutique pour une utilisation en tant que médicament dans l'induction de la régénération osseuse chez un hôte comprenant :
un anticorps ou une partie de celui-ci se liant à l'antigène, qui se lie spécifiquement à l'antigène de la protéine morphogénique osseuse de type 2 (BMP-2),
dans laquelle l'anticorps se lie à un épitope éloigné d'un domaine de liaison au récepteur BMP-2 et
dans laquelle le complexe anticorps/BMP-2 induit la différenciation ostéogénique.

2. Composition pour une utilisation selon la revendication 1, dans laquelle le complexe anticorps/BMP-2 induit la différenciation ostéogénique par transduction de signaux.

3. Composition pour une utilisation selon la revendication 1, dans laquelle le complexe anticorps/BMP-2 se lie à un récepteur de BMP sur une cellule ostéoprogénitrice.

4. Composition pour une utilisation selon la revendication 1, dans laquelle un complexe de l'anticorps et de la BMP-2 endogène se lie à un récepteur de BMP sur une cellule ostéoprogénitrice et favorise la formation osseuse.

5. Composition pour une utilisation selon la revendication 1, dans laquelle l'anticorps augmente la disponibilité de la protéine BMP-2 endogène entourant la cellule.

6. Anticorps pour une utilisation en tant que médicament dans l'induction de la régénération osseuse ayant des caractéristiques telles que :
l'anticorps se lie à un épitope de la protéine morphogénique osseuse endogène de type 2 (BMP-2),
dans lequel l'anticorps se lie à l'épitope de BMP-2 éloigné des domaines de liaison du récepteur de BMP-2, et
dans lequel un complexe constitué de l'anticorps et de la BMP-2 endogène favorise la formation osseuse impliquée dans l'ostéo-intégration ou l'ostéogenèse ; et facultativement au moins une partie d'une région constante est humanisée, ou au moins une partie de la chaîne lourde est humanisée.

7. Anticorps pour une utilisation selon la revendication 6, dans lequel la liaison de l'anticorps génère la transduction de signaux intracellulaires.

8. Anticorps pour une utilisation selon la revendication 6, qui est entièrement humain.

9. Implant médical comprenant :
une structure d'implant biocompatible adaptée pour accueillir une structure squelettique du corps ;
un anticorps déposé sur la structure d'implant, dans lequel l'anticorps se lie spécifiquement à un épitope de la protéine morphogénique osseuse, se lie à l'épitope de BMP-2 éloigné des domaines de liaisons du récepteur de BMP-2 et le complexe anticorps/BMP-2 induit des différenciations ostéogéniques, et facultativement dans lequel l'anticorps est déposé sur l'implant avec un lieur synthétique, ou l'anticorps est déposé sur la structure d'implant par adsorption.

10. Implant selon la revendication 9, dans lequel un complexe anticorps/BMP-2 induit une différenciation ostéogénique par transduction de signaux.

11. Implant selon la revendication 9, dans lequel le complexe anticorps/BMP-2 se lie à un récepteur de BMP-2 sur une cellule ostéoprogénitrice.

12. Implant selon la revendication 9, dans lequel le complexe anticorps/BMP-2 favorise la formation osseuse.

13. Implant selon la revendication 9, dans lequel l'anticorps augmente la disponibilité de la protéine BMP-2 endogène entourant la cellule.
